(19) Europäisches Patentamt
European Patent Office
Office européen des brevets

(11) EP 3 655 397 B1

(12) **FASCICULE DE BREVET EUROPEEN**

(45) Date de publication et mention de la délivrance du brevet:
**01.09.2021 Bulletin 2021/35**

(21) Numéro de dépôt: **18743788.4**

(22) Date de dépôt: **20.07.2018**

(51) Int Cl.:
***C07D 257/02*** *(2006.01)* ***A61P 35/00*** *(2006.01)*
***A61K 49/00*** *(2006.01)* ***A61K 51/00*** *(2006.01)*

(86) Numéro de dépôt international:
**PCT/EP2018/069784**

(87) Numéro de publication internationale:
**WO 2019/016377 (24.01.2019 Gazette 2019/04)**

(54) **LIGANDS MACROCYCLIQUES LIPOPHILES, LEURS COMPLEXES AINSI QUE LEURS UTILISATIONS MÉDICALES**

LIPOPHILE MAKROCYCLISCHE LIGANDEN, KOMPLEXE DAVON UND VERWENDUNGEN DAVON

LIPOPHILIC MACROCYCLIC LIGANDS, COMPLEXES THEREOF, AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.07.2017 FR 1756932**

(43) Date de publication de la demande:
**27.05.2020 Bulletin 2020/22**

(73) Titulaire: **Guerbet**
**93420 Villepinte (FR)**

(72) Inventeurs:
- **ROUSSEAUX, Olivier**
**60300 Senlis (FR)**
- **FOUGERE, Olivier**
**60128 Mortefontaine (FR)**
- **CATOEN, Sarah**
**93190 Livry Gargan (FR)**

(74) Mandataire: **Lavoix**
**2, place d'Estienne d'Orves**
**75441 Paris Cedex 09 (FR)**

(56) Documents cités:
**EP-A1- 0 922 700**
**WO-A1-03/009874**
**WO-A1-2006/002874**
**WO-A2-03/074523**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 655 397 B1

## Description

La présente invention concerne de nouveaux ligands macrocycliques lipophiles ainsi que leurs complexes, notamment radioactifs, et leurs utilisations en imagerie médicale et/ou en thérapie, notamment en radiologie interventionnelle.

Le besoin de traitements ciblés et personnalisés en oncologie conduit à développer des stratégies thérapeutiques nouvelles basées sur des outils de détection précoces associés à des traitements vectorisés plus spécifiques et plus efficaces.

La radiologie interventionnelle est un axe très prometteur de la médecine individualisée. Elle permet de combiner dans une même séquence un diagnostic précis de la lésion ou tumeur et/ou son traitement instantané, guidé et contrôlé par l'image. Elle est décrite comme une chirurgie minimalement invasive et peut être de ce fait réalisée en ambulatoire, ce qui permet d'économiser de nombreuses et onéreuses journées d'hospitalisation pour une efficacité souvent comparable à la chirurgie conventionnelle. La radiologie interventionnelle peut donc représenter une alternative ou un complément au traitement chirurgical conventionnel.

La radiologie interventionnelle permet d'accéder à une lésion ou tumeur située à l'intérieur de l'organisme pour effectuer un acte diagnostique (prélèvement par exemple) ou thérapeutique. L'imagerie par fluoroscopie, échographie, scanner ou IRM permet un repérage, un guidage et un contrôle optimal du geste médical.

Par ailleurs, les demandes WO 03/074523 et EP 0 922 700 décrivent respectivement des oligomères de chélates de gadolinium, leur application comme produit de contraste en imagerie par résonance magnétique et leurs intermédiaires de synthèse, ainsi que des chélates métalliques de dérivés macrocycliques polyaminocarboxyliques et leur application à l'imagerie diagnostique.

Il existe donc un besoin pour de nouvelles molécules utilisables en imagerie médicale et/ou en thérapie, en particulier en radiologie interventionnelle. Plus particulièrement, il existe un besoin pour des ligands permettant de complexer des éléments chimiques, en particulier des métaux, de façon à obtenir des complexes utilisables en imagerie médicale et/ou en thérapie, en particulier en radiologie interventionnelle. Il existe également un besoin pour des ligands permettant de complexer des éléments chimiques qui puissent être formulés de façon stable dans des compositions adaptées à l'imagerie médicale et/ou en thérapie.

De tels ligands doivent notamment être stables et complexer de façon suffisamment forte les métaux pour que ceux-ci atteignent leur cible et ne diffusent pas dans d'autres organes ou tissus sensibles comme les os, les poumons et les reins. Ces ligands doivent en particulier permettre de stabiliser les éléments radioactifs dans les formulations pharmaceutiques voulues, en évitant la diffusion de la radioactivité dans tout l'organisme une fois administrés.

La présente invention a pour but de fournir de nouveaux ligands permettant de complexer des éléments chimiques, en particulier des radioéléments.

La présente invention a également pour but de fournir de nouveaux complexes, en particulier des complexes radioactifs.

La présente invention a pour but de fournir des ligands et/ou des complexes particulièrement utiles en imagerie médicale et/ou en thérapie, notamment dans le traitement des cancers.

La présente invention a également pour but de fournir une composition pharmaceutique stable comprenant des complexes permettant l'imagerie médicale, le ciblage et/ou le traitement de cancers.

La présente invention a pour but de fournir une composition pharmaceutique stable permettant la vectorisation de complexes selon l'invention, de façon sûre et efficace pour les patients.

La présente invention concerne un composé de formule générale (I) suivante :

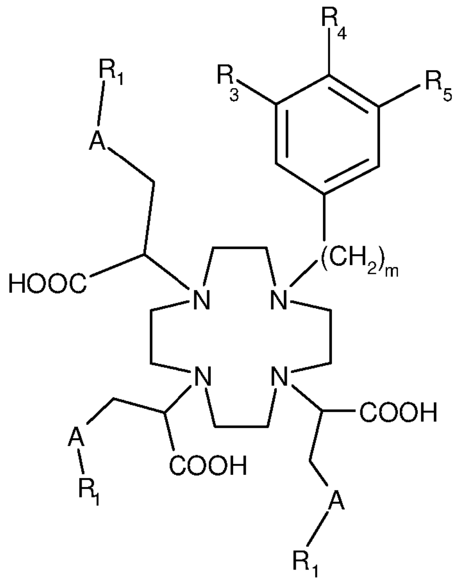

(I)

dans laquelle :

- $R_1$ est un méthyle ou un $(C_6\text{-}C_{10})$aryle ;
- $R_3$, $R_4$ et $R_5$ sont choisis indépendamment les uns des autres parmi le groupe constitué de : H, $(C_1\text{-}C_{20})$alkyle, $(C_2\text{-}C_{20})$alcényle, $(C_2\text{-}C_{20})$alcynyle, $(C_6\text{-}C_{10})$aryle, $(C_1\text{-}C_{20})$alkylène-$(C_6\text{-}C_{10})$aryle, $(C_2\text{-}C_{20})$alcénylène-$(C_6\text{-}C_{10})$aryle et $(C_2\text{-}C_{20})$alcynylène-$(C_6\text{-}C_{10})$aryle ;
  lesdits groupements alkyle, alcényle, alcynyle, alkylène, alcénylène et alcynylène des radicaux $R_3$, $R_4$ et $R_5$ pouvant éventuellement comprendre un ou plusieurs $(C_6\text{-}C_{10})$arylène(s) et/ou un ou plusieurs $(C_5\text{-}C_{10})$cycloalkylène(s) dans leur chaîne; et
  lesdits groupements alkyle, alcényle, alcynyle, aryle, alkylène, alcénylène et alcynylène des radicaux $R_3$, $R_4$ et $R_5$ étant éventuellement substitué(s) par un ou plusieurs substituant(s) choisis parmi le groupe constitué de : halogène, halogéno$(C_1\text{-}C_{20})$alkyle, $(C_1\text{-}C_{20})$alkyle, $(C_2\text{-}C_{20})$alcényle, $(C_2\text{-}C_{20})$alcynyle ; lesdits groupements alkyle, alcényle et alcynyle, pouvant éventuellement comprendre un ou plusieurs $(C_6\text{-}C_{10})$arylène(s) dans leur chaîne ;
- A est un groupement $-(CH_2)_n-$ pouvant éventuellement comprendre un ou plusieurs $(C_6\text{-}C_{10})$arylène(s) dans sa chaîne ;
- n est un nombre entier allant de 0 à 15, de préférence de 0 à 10 ; et
- m est un nombre entier allant de 1 à 10 ;
  ou l'un de ses sels pharmaceutiquement acceptables ou l'un de ses isomères optiques ou l'un de ses isomères géométriques ou l'un de ses tautomères ou l'un de ses solvates.

Les inventeurs ont mis au point de nouveaux complexes ligands-métal (complexes appelés également chélates) à partir du macrocycle cyclène 1,4,7,10-tetraazacyclododecane, substitué par trois bras identiques sur trois des atomes d'azote, ainsi que par un bras lipophile comprenant au moins un cycle phényle sur le dernier atome d'azote. Le macrocycle cyclène est de formule suivante :

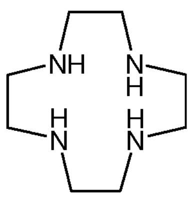

De façon surprenante, les complexes selon l'invention présentent une bonne stabilité thermodynamique ainsi qu'une bonne inertie cinétique. De plus, de façon tout aussi surprenante, les inventeurs ont découvert que les complexes selon l'invention pouvaient être solubilisés dans une huile iodée telle que LIPIODOL®, une huile iodée fabriquée et commercialisée par la société Guerbet et qui est constituée par des esters éthyliques des acides gras iodés de l'huile d'œillette. Ainsi, les complexes selon l'invention solubilisés dans une huile iodée telle que LIPIODOL® peuvent être

vectorisés notamment vers le foie et peuvent permettre de visualiser et/ou traiter les cancers, par exemple les cancers du foie.

Ces complexes présentent également un bon rendement radiochimique de l'extraction dans une huile iodée telle que LIPIODOL®. Ils présentent en particulier une bonne incorporation de la radioactivité dans une huile iodée telle que LIPIODOL® et une bonne stabilité de la solution radioactive de LIPIODOL® lors de tests *in vitro.*

En particulier, la combinaison des propriétés de vectorisation de LIPIODOL®, d'efficacité thérapeutique des radioéléments, et la bonne tolérance de ces produits permettent de proposer un traitement thérapeutique des cancers sûr et plus facile à mettre en œuvre.

La vectorisation des complexes selon l'invention par une huile iodée telle que LIPIODOL® permet notamment d'éviter une mauvaise délivrance des complexes en diminuant le risque d'effets indésirables dans les organes sains, en particulier le foie sain ou dans les organes extra-hépatiques, et permet d'atteindre la dose de radioactivité efficace dans la tumeur.

Plus particulièrement, cette vectorisation facilite le travail du radiologue interventionnel au moment de l'injection des complexes selon l'invention. Par exemple, lors d'une injection intra-artérielle suivie sous fluoroscopie, le geste du radiologue sera plus précis et plus sûr en permettant un ajustement de la vitesse de délivrance des complexes en fonction de la capture par la tumeur des complexes selon l'invention.

**Définitions**

On entend par « ligand », un composé capable de complexer un élément chimique tel qu'un métal, de préférence un radioélément. Selon un mode de réalisation, les ligands au sens de l'invention sont sous forme anionique et peuvent complexer des radioéléments sous forme cationique, par exemple des cations métalliques au degré d'oxydation (III). Selon la présente invention, les composés de formule (I) sont des ligands.

On entend par « radioélément », tout radioisotope connu d'un élément chimique, qu'il soit naturel ou produit artificiellement. Selon un mode de réalisation, le radioélément est choisi parmi les radioisotopes de l'yttrium, des terres rares et des lanthanides.

Par « terres rares » sont désignés les atomes choisis parmi le groupe constitué du scandium Sc, de l'yttrium Y, et des lanthanides.

Par « lanthanides » sont désignés les atomes choisis parmi le groupe constitué de : La, Ce, Pr, Nd, Pm, Sm, Eu, Gd, Tb, Dy, Ho, Er, Tm, Yb et Lu (soit Lanthane, Cérium, Praséodyme, Néodyme, Prométhium, Samarium, Europium, Gadolinium, Terbium, Dysprosium, Holmium, Erbium, Thulium, Ytterbium et Lutécium).

On entend par « complexe », l'association d'un ligand tel que défini ci-dessus avec un élément chimique, de préférence un radioélément tel que défini ci-dessus. Le terme « complexe » étant synonyme de « chélate ».

On entend par « taux d'extraction » ou « rendement d'extraction », la quantité de radioactivité transférée d'un milieu, par exemple un milieu polaire, comprenant un complexe radioactif selon l'invention, de préférence d'un milieu réactionnel de synthèse d'un complexe radioactif selon l'invention vers une phase huileuse, préférentiellement une huile iodée et plus préférentiellement Lipiodol®. Cette quantité est exprimée en pourcentage de la radioactivité initialement engagée exprimée en curie ou en becquerel. Par « milieu réactionnel de synthèse d'un complexe radioactif selon l'invention », on entend par exemple un mélange de tampon acétate et d'éthanol.

On entend par « rendement de radiomarquage », la quantité de radioactivité se retrouvant sous la forme d'un complexe après l'étape de radiomarquage d'un ligand. Cette quantité est exprimée en pourcentage de la radioactivité initialement engagée exprimée en curie ou en becquerel.

La « stabilité thermodynamique » représente l'affinité du ligand pour un élément donné, en particulier un métal donné. Il s'agit de la constante d'équilibre de la réaction de complexation :

$$\text{Métal} + \text{Ligand} \rightleftarrows \text{Complexe}$$

dont les constantes sont les suivantes :

Dissociation (le complexe se dissociant en un ligand et un métal) :

$$K_D = \frac{[Métal]\ [Ligand]}{[Complexe]}$$

Association (le ligand et le métal s'associant pour former un complexe):

$$K_A = \frac{[Complexe]}{[Métal]\,[Ligand]} \text{ avec } K_D = \frac{1}{K_A}$$

Les valeurs sont en général exprimées sous forme de logarithme décimal $logK_A$ ou $-logK_D$. Selon un mode de réalisation, les complexes selon l'invention sont de forte affinité. Selon un mode de réalisation, les complexes selon l'invention ont une constante thermodynamique d'équilibre au moins égale à 16 ($LogK_A$ au moins égal à 16).

Les complexes formés selon la réaction d'équilibre décrite ci-dessus sont susceptibles de se dissocier sous l'action de différents facteurs (pH, présence de métaux ou ligands compétiteurs). Cette dissociation peut avoir des conséquences importantes dans le cadre de l'utilisation des complexes en médecine humaine car elle entraine une libération du métal dans l'organisme. Afin de limiter ce risque, des complexes à dissociation lente sont recherchés, c'est-à-dire des complexes ayant une bonne inertie cinétique. L'inertie cinétique peut être déterminée par des tests de dissociation en milieu acide. Ces expériences conduisent à la détermination pour chaque complexe d'un temps de demi-vie ($T_{1/2}$) dans des conditions définies.

Dans le contexte de l'invention, le terme « traiter », « traitement » ou « traitement thérapeutique » signifie inverser, soulager, inhiber la progression de, du trouble ou l'affection auquel ce terme est applicable, ou un ou plusieurs symptôme(s) d'un tel trouble.

Le terme « imagerie médicale » désigne les moyens d'acquisition et de restitution d'images du corps humain ou animal à partir de différents phénomènes physiques tels que l'absorption des rayons X, la résonance magnétique nucléaire, la réflexion d'ondes ultrasons ou la radioactivité. Selon un mode de réalisation, le terme « imagerie médicale » se réfère à l'imagerie par rayons X, l'IRM (Imagerie par Résonance Magnétique), la tomographie d'émission monophotonique (TEMP ou SPECT pour « Single Photon Emission Computed Tomography »), la tomoscintigraphie par émission de positons (TEP) et la luminescence. De préférence, la méthode d'imagerie médicale est l'imagerie par rayons X. Selon un mode de réalisation particulier, la méthode d'imagerie médicale est l'IRM si le complexe selon l'invention comprend du Gd(III), SPECT si le complexe selon l'invention comprend un émetteur gamma et TEP si le complexe selon l'invention comprend un émetteur béta+.

La capacité des agents de contraste à accélérer les vitesses de relaxation 1/T1 et 1/T2 des protons de l'eau est mesurée par une grandeur, la relaxivité. On définit notamment la relaxivité (r) d'un agent de contraste comme la vitesse de relaxation, normalisée par la concentration de l'agent de contraste.

Le terme « $(C_1-C_{20})$alkyle » désigne des hydrocarbures aliphatiques saturés, qui peuvent être linéaires ou ramifiés et comprennent de 1 à 20 atomes de carbone. De préférence, les alkyles comprennent de 1 à 10 atomes de carbone, voire de 1 à 5 atomes de carbone. Par « ramifié », on entend qu'un groupement alkyle est substitué sur la chaîne alkyle principale.

Le terme « $(C_1-C_{20})$alkylène » désigne un radical alkyle tel que défini ci-dessus, divalent.

Le terme « $(C_2-C_{20})$alcène » désigne un alkyle tel que défini ci-dessus, comprenant au moins une double liaison carbone-carbone.

Le terme « $(C_2-C_{20})$alcénylène » désigne un alkyle tel que défini ci-dessus, comprenant au moins une double liaison carbone-carbone et divalent.

Le terme « $(C_2-C_{20})$alcyne » désigne un alkyle tel que défini ci-dessus, comprenant au moins une triple liaison carbone-carbone.

Le terme « $(C_2-C_{20})$alcynylène » désigne un alkyle tel que défini ci-dessus, comprenant au moins une triple liaison carbone-carbone et divalent.

Le terme « $(C_6-C_{10})$aryle » désigne des composés aromatiques hydrocarbonés monocycliques, bicycliques ou tricycliques, en particulier le phényle et le naphtyle.

Le terme « arylène » désigne un aryle tel que défini ci-dessus, divalent, en particulier le phénylène et le naphtylène.

Le terme « $(C_5-C_{10})$cycloalkylène » désigne un cycloalkyle comprenant de 5 à 10 atomes de carbone, monocyclique ou bicyclique, divalent. Parmi les cycloalkylènes, peuvent être cités le cyclopentylène ou le cyclohexylène.

Selon un mode de réalisation, « halogène » désigne F, Cl, Br, I, At et leurs isotopes, de préférence F, Cl, Br, I et leurs isotopes. Selon un mode de réalisation particulier, l'halogène est un atome de fluor.

**Les huiles iodées**

On entend désigner par le terme « acide gras » des acides carboxyliques aliphatiques saturés ou insaturés présentant une chaîne carbonée d'au moins 4 atomes de carbone. Les acides gras naturels possèdent une chaîne carbonée de 4 à 28 atomes de carbone (généralement un nombre pair). On parle d'« acide gras à longue chaîne » pour une longueur de 14 à 22 carbones et à très longue chaîne s'il y a plus de 22 carbones. On parle au contraire d'« acide gras à courte chaîne » pour une longueur de 4 à 10 carbones, notamment 6 à 10 atomes de carbone, en particulier 8

ou 10 atomes de carbone. L'homme du métier connaît la nomenclature associée et en particulier utilise :

- Ci-Cp pour désigner une fourchette d'acides gras en Ci à Cp
- Ci+Cp, le total des acides gras en Ci et des acides gras en Cp

Par exemple :

- les acides gras de 14 à 18 atomes de carbone s'écrivent « acides gras en C14-C18 »
- le total des acides gras en C16 et des acides gras en C18 s'écrit C16 +C18.
- pour un acide gras saturé, un homme du métier utilisera la nomenclature suivante Ci : 0, où i est le nombre d'atomes de carbone de l'acide gras. L'acide palmitique sera par exemple désigné par la nomenclature (C16 :0).
- pour un acide gras insaturé, un homme du métier utilisera la nomenclature suivante Ci : x n-N où N sera la position de la double liaison dans l'acide gras insaturé en partant du carbone opposé au groupe acide, i est le nombre d'atomes de carbone de l'acide gras, x est le nombre de double liaisons (insaturations) de cet acide gras. L'acide oléique, sera par exemple désigné par la nomenclature (C18 :1 n-9).

De façon avantageuse, l'huile iodée selon l'invention comprend ou est constituée de dérivés d'acides gras iodés, préférentiellement d'esters éthyliques d'acides gras iodés, plus préférentiellement d'esters éthyliques d'acides gras iodés d'huile d'œillette, d'huile d'olive, d'huile de graines de colza, d'huile d'arachide, d'huile de graines de soja ou d'huile de noix, encore plus préférentiellement d'esters éthyliques d'acides gras iodés d'huile d'œillette ou d'huile d'olive. Plus préférentiellement, l'huile iodée selon l'invention comprend ou est constituée d'esters éthyliques d'acides gras iodés d'huile d'œillette (aussi appelée pavot noir ou *Papaver somniferum var. nigrum*). L'huile d'œillette, aussi appelée huile de graines de pavot ou huile de graines d'œillette, contient préférentiellement plus de 80% d'acides gras insaturés (en particulier de l'acide linoléique (C18 :2 n-6) et de l'acide oléique (C18 :1 n-9)) dont au moins 70% d'acide linoléique et au moins 10% d'acide oléique. L'huile iodée est obtenue à partir de la complète iodation d'une huile telle que l'huile d'œillette dans des conditions permettant une liaison d'un atome d'iode pour chaque double liaison des acides gras insaturés (Wolff et al. 2001, Medicine 80, 20-36) suivie d'une trans-estérification.

L'huile iodée selon l'invention contient préférentiellement de 29 à 53% (m/m), plus préférentiellement 37% à 39% (m/m) d'iode.

Comme exemples d'huiles iodées peuvent être cités le Lipiodol®, le Brassiodol® (issu de l'huile de graines de colza (*Brassica compestis*), le Yodiol® (issu de l'huile d'arachide), l'Oriodol® (issu de l'huile d'œillette mais sous forme de triglycérides d'acides gras), le Duroliopaque® (issu de l'huile d'olive).

Préférentiellement, l'huile iodée est Lipiodol®, une huile iodée utilisée comme produit de contraste et dans certaines procédures de radiologie interventionnelle. Cette huile est un mélange d'esters éthyliques d'acides gras iodés et non-iodés d'huile de graines d'œillette. Elle consiste majoritairement (en particulier, plus de 84%) en un mélange d'esters éthyliques d'acides gras iodés à longue-chaine (en particulier des acides gras en C18) issus de l'huile de graines d'œillette, préférentiellement en un mélange de monoiodostéarate d'éthyle et de diiodostéarate d'éthyle. L'huile iodée peut aussi être une huile à base d'ester éthylique monoiodé d'acide stéarique (C18 :0) issu de l'huile d'olive. Un produit de ce type, s'appelant Duroliopaque® était commercialisé il y a quelques années.

Le terme « LIPIODOL» se réfère à une huile iodée et de manière préférentielle à la spécialité pharmaceutique LIPIODOL®, solution injectable fabriquée et commercialisée par Guerbet et constituée par des esters éthyliques des acides gras iodés de l'huile d'œillette. LIPIODOL® est un produit notamment utilisé pour la visualisation, la localisation et/ou la vectorisation au cours de la chimioembolisation transartérielle du carcinome hépatocellulaire au stade intermédiaire, chez l'adulte ainsi que pour le diagnostic par voie artérielle hépatique sélective de l'extension hépatique des lésions malignes hépatiques ou non.

Les caractéristiques principales de Lipiodol® sont les suivantes :

| **Composés** | **Proportions dans le mélange d'acide gras** |
|---|---|
| Palmitate d'éthyle (Ethyl C16 :0) | 4,6 à 6,7% (m/m), préférentiellement 4,8% (m/m) |
| Stéarate d'éthyle (Ethyl C18 :0) | 0,8 à 1,9% (m/m), préférentiellement 1,2% (m/m) |
| Monoiodostéarate d'éthyle | 11,3 à 15,3% (m/m), préférentiellement 13,4% (m/m) |
| Diiodostéarate d'éthyle | 73,5 à 82,8% (m/m), préférentiellement 78,5% (m/m) |

| Autres caractéristiques du Lipiodol® : | |
|---|---|
| Iode | 37% à 39% (m/m) (soit 480 mg/ml) |
| Viscosité<br>à 37°C<br>à 20°C | <br>25 mPa.s<br>50 mPa.s |
| Densité | 1,268 - 1,290 g/cm$^3$ à 20°C, préférentiellement 1,28 |

**Composés de formule générale (I)**

Les composés de formule générale (I) peuvent avoir des centres de chiralité et peuvent être sous forme racémique ou énantiomérique. Les composés de formule générale (I) sont compris sous leurs différentes formes isomériques incluant les énantiomères, les diastéréoisomères ou les mélanges racémiques des couples d'énantiomères ou les mélanges de diastéréoisomères.

Les modes de réalisation qui suivent peuvent être considérés indépendamment les uns des autres ou combinés entre eux.

Selon un mode de réalisation, les composés de formule générale (I) sont sous forme de sel, de préférence sous forme de sel pharmaceutiquement acceptable.

Par « sel pharmaceutiquement acceptable », on désigne notamment des sels permettant de conserver les propriétés et l'efficacité biologique des composés selon l'invention. Des exemples de sels pharmaceutiquement acceptables se trouvent dans Berge, et al. ((1977) J. Pharm. Sd, vol. 66, 1). Par exemple, les composés de formule générale (I) sont sous forme de sel de sodium ou de méglumine (1-Deoxy-1-(methylamino)-D-glucitol ou *N*-Methyl-D-glucamine).

L'invention concerne également les isomères optiques (énantiomères), géométriques (cis/trans ou Z/E), les tautomères et les solvates tels que les hydrates des composés de formule (I).

Selon un mode de réalisation, lorsque $R_1$ est un méthyle, alors n est un nombre entier allant de 4 à 8. Selon un mode de réalisation, lorsque $R_1$ est un méthyle, alors n est un nombre entier allant de 4 à 8 et lorsque que $R_1$ est un $(C_6\text{-}C_{10})$aryle, alors n est un nombre entier allant de 0 à 6, de préférence de 0 à 5. Selon un mode de réalisation particulier, lorsque $R_1$ est un méthyle, alors n est un nombre entier égal à 4, 6 ou 8.

Selon un mode de réalisation, n est un nombre entier allant de 1 à 15, de préférence de 1 à 10, plus préférentiellement de 4 à 8, par exemple égal à 4, 6 ou 8. Selon un mode de réalisation, m est un nombre entier allant de 1 à 5, de préférence de 1 à 3, par exemple égal à 1.

Selon un mode de réalisation, $R_1$ est un méthyle ou un phényle. Selon un mode de réalisation, le groupement A et/ou les radicaux $R_3$, $R_4$ et/ou $R_5$ comprennent au maximum 3 groupements arylènes dans leur(s) chaîne(s).

Selon un mode de réalisation, A est un groupement $-(CH_2)_n$.

Selon un mode de réalisation particulier, le $(C_6\text{-}C_{10})$aryle est un phényle. Selon un mode de réalisation, $R_3$, $R_4$ et $R_5$ sont choisis indépendamment les uns des autres parmi le groupe constitué de : H, $(C_1\text{-}C_{20})$alkyle, $(C_2\text{-}C_{20})$alcényle, $(C_2\text{-}C_{20})$alcynyle, $(C_6\text{-}C_{10})$aryle, $(C_1\text{-}C_{20})$alkylène-$(C_6\text{-}C_{10})$aryle, $(C_2\text{-}C_{20})$alcénylène-$(C_6\text{-}C_{10})$aryle et $(C_2\text{-}C_{20})$alcynylène-$(C_6\text{-}C_{10})$aryle ;
lesdits groupements alkyle, alcényle, alcynyle, alkylène, alcénylène et alcynylène des radicaux $R_3$, $R_4$ et $R_5$ pouvant éventuellement comprendre un ou plusieurs $(C_6\text{-}C_{10})$arylène(s) dans leur chaîne ;
lesdits groupements alkyle et/ou aryle étant éventuellement substitué(s) par un ou plusieurs substituant(s) choisis parmi les halogènes, les $(C_1\text{-}C_{20})$alkyles ou les halogéno$(C_1\text{-}C_{20})$alkyles.

Selon un mode de réalisation, $R_3$ et $R_5$ sont choisis indépendamment l'un de l'autre parmi H ou $(C_1\text{-}C_{20})$alkyle, ledit groupement alkyle étant éventuellement substitué par un ou plusieurs substituant(s) choisis parmi les halogènes, de préférence le fluor.

De préférence, $R_3$ et $R_5$ sont choisis indépendamment l'un de l'autre parmi H, tert-butyle ou $CF_3$. Selon un mode de réalisation, $R_3$ et $R_5$ sont identiques, de préférence sont H ou un tert-butyle, plus préférentiellement H.

Selon un mode de réalisation, $R_4$ est choisi parmi le groupe constitué de : H, $(C_1\text{-}C_{10})$alkyle, $(C_6\text{-}C_{10})$aryle, $(C_1\text{-}C_{10})$alkylène-$(C_6\text{-}C_{10})$aryle et $(C_2\text{-}C_{10})$alcénylène-$(C_6\text{-}C_{10})$aryle et $(C_2\text{-}C_{10})$alcynylène-$(C_6\text{-}C_{10})$aryle ;
lesdits groupements alkyle, aryle, alkylène et alcénylène étant éventuellement substitué(s) par un ou plusieurs substituant(s) choisis parmi les halogènes ou les halogéno$(C_1\text{-}C_{20})$alkyles.

Selon un mode de réalisation, $R_3$, $R_4$ et $R_5$ sont choisis parmi le groupe constitué de : H, $(C_1\text{-}C_{20})$alkyle, $(C_6\text{-}C_{10})$aryle et $(C_1\text{-}C_{20})$alkylène-$(C_6\text{-}C_{10})$aryle ;
lesdits groupements alkyle, aryle et alkylène des radicaux $R_3$, $R_4$ et $R_5$ étant éventuellement substitué(s) par un ou plusieurs groupement(s) $CF_3$.

Selon un mode de réalisation, $R_4$ est choisi parmi le groupe constitué de : H, $(C_1\text{-}C_{20})$alkyle, phényle et

$(C_1-C_{20})$alkylène-phényle. Selon un autre mode de réalisation, $R_4$ est choisi parmi le groupe constitué de : H, tert-butyle, phényle, $-CH_2-CH_2$-phényle, et-$(CH_2)_7-CH_3$.

Selon un mode de réalisation, les composés selon l'invention sont de formule générale (I-1) suivante :

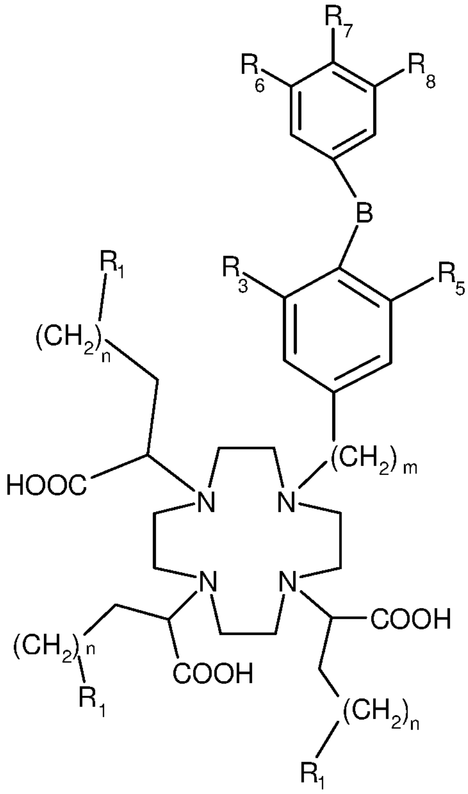

(I-1)

dans laquelle :

- $R_1$, $R_3$, $R_5$, n et m sont tels que définis à la revendication 1, avec n étant de préférence égal à 6 ou égal à 8;
- B est une liaison, un $(C_1-C_{20})$alkylène, un $(C_2-C_{20})$alcénylène ou un $(C_2-C_{20})$alcynylène ; et
- $R_6$, $R_7$ et $R_8$ sont choisis, indépendamment les uns des autres, parmi H et $(C_1-C_{20})$alkyle. De préférence, $R_6$ et $R_8$ sont H et $R_7$ est $(C_1-C_{20})$alkyle.

Selon un mode particulier, les composés selon l'invention sont de formule générale (I-1-1) suivante :

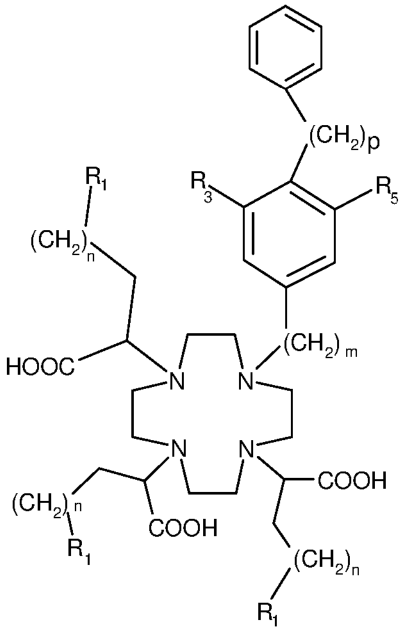

(I-1-1)

dans laquelle $R_1$, $R_3$, $R_5$, n et m sont tels que définis ci-dessus, avec n étant de préférence égal à 6, et p est un nombre entier allant de 0 à 10, de préférence allant de 0 à 5, par exemple égal à 0, 1, 2 ou 3.

Selon un mode de réalisation, les composés selon l'invention sont de formule générale (I-2) suivante :

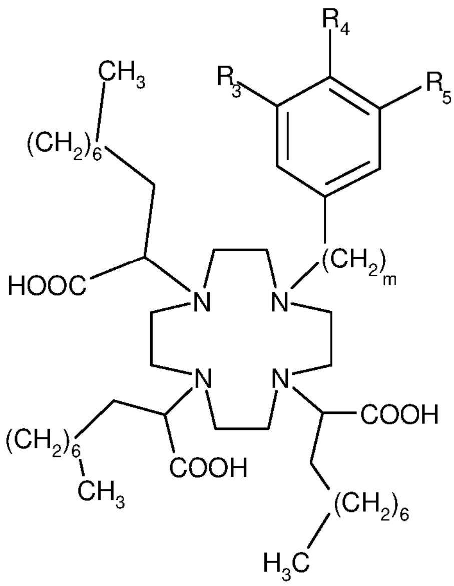

(I-2)

dans laquelle $R_3$, $R_4$, $R_5$ et m sont tels que définis ci-dessus.

Selon un mode de réalisation, les composés selon l'invention sont de formule générale (I-3) suivante :

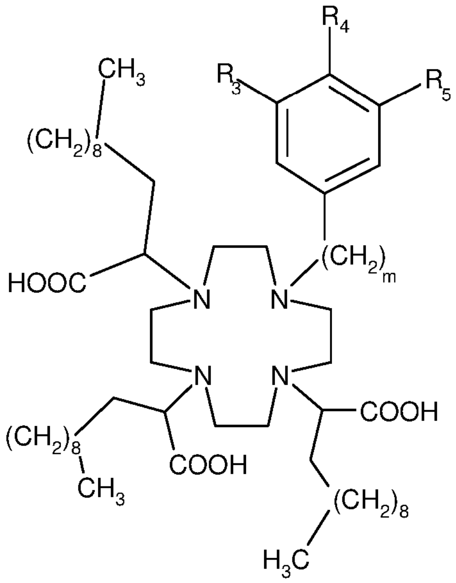

(I-3)

dans laquelle $R_3$, $R_4$, $R_5$ et m sont tels que définis ci-dessus.

Selon un mode de réalisation, les composés selon l'invention sont de formule générale (I-4) suivante :

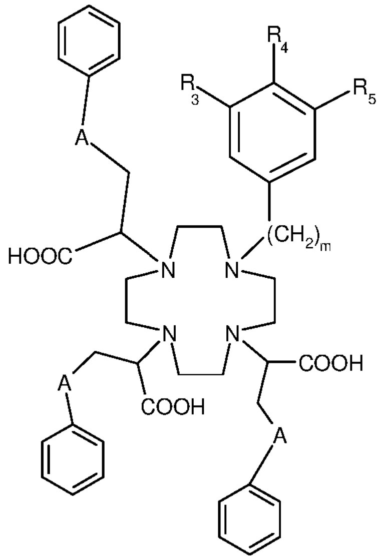

(I-4)

dans laquelle $R_3$, $R_4$, $R_5$, A et m sont tels que définis ci-dessus.

La présente invention concerne également un composé choisi parmi le groupe constitué des composés suivants :

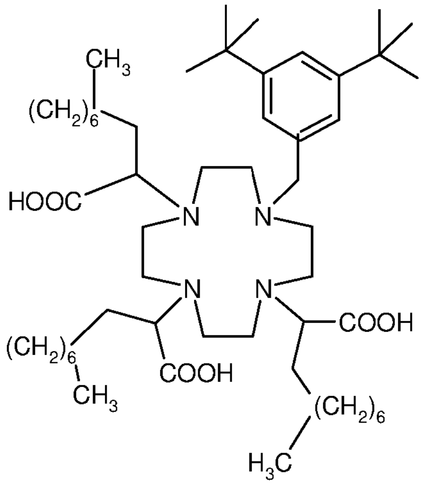

G3

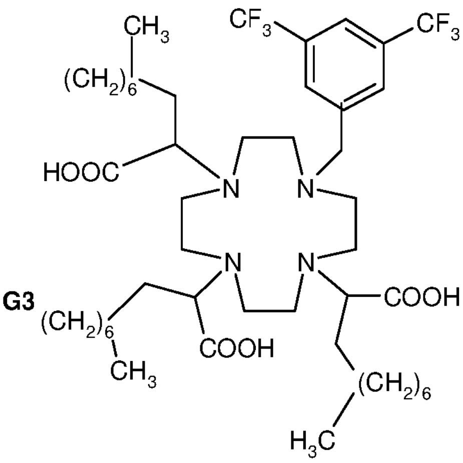

G4

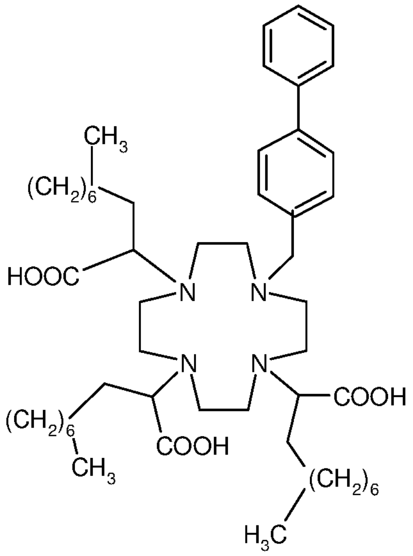

G6

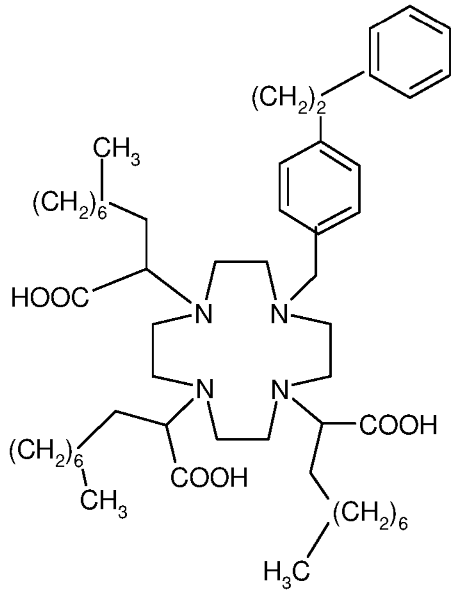

G5

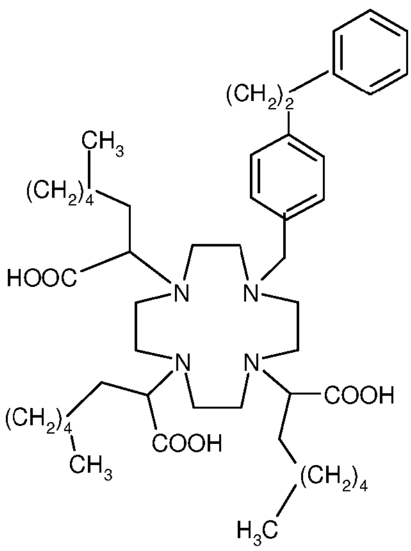

G9

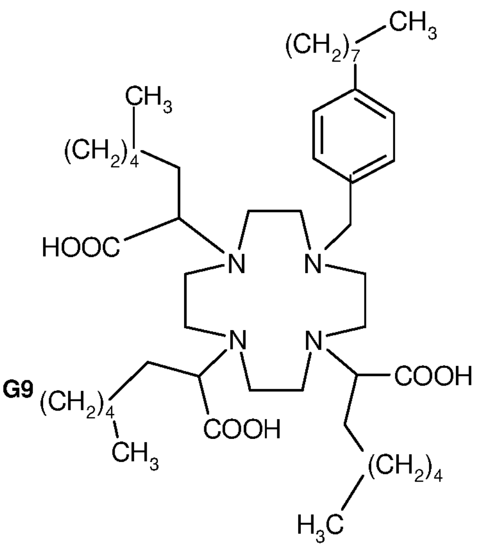

G8

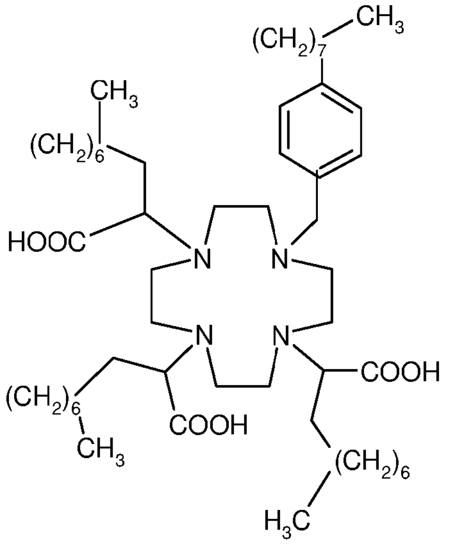

G7

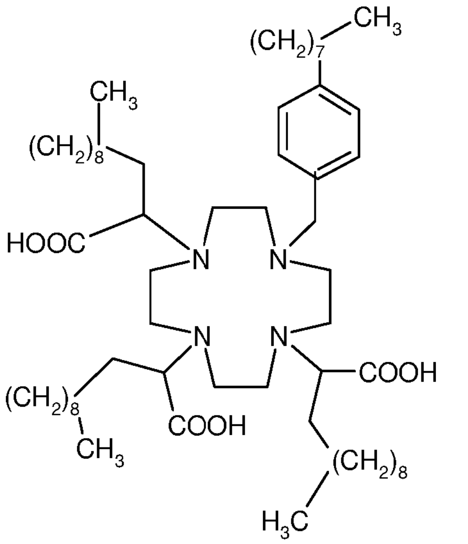

G2

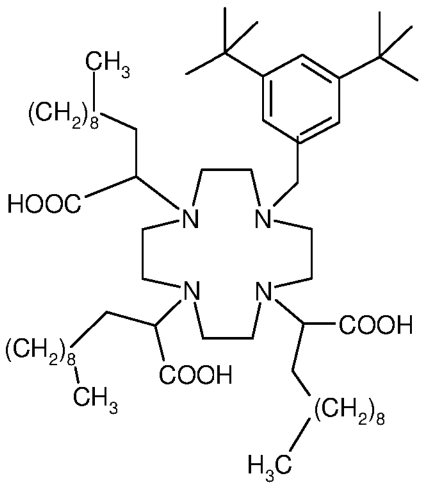

G1

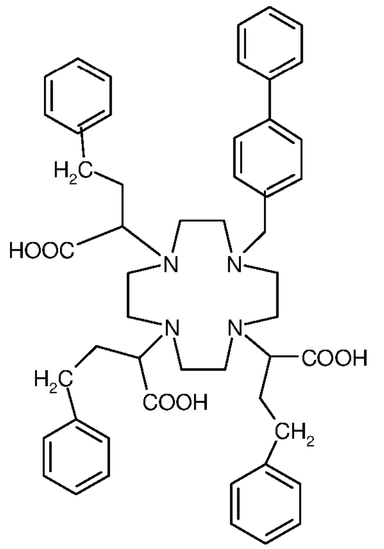

G10

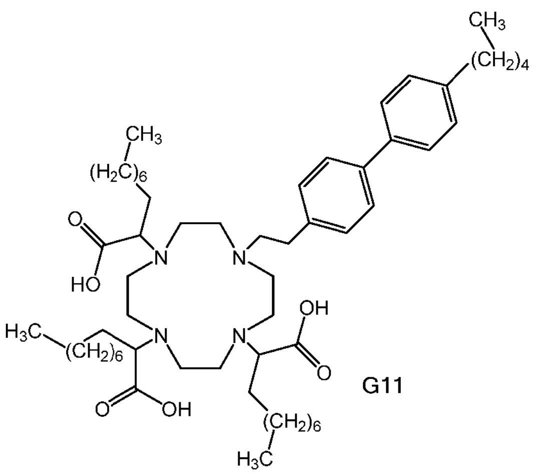

G11

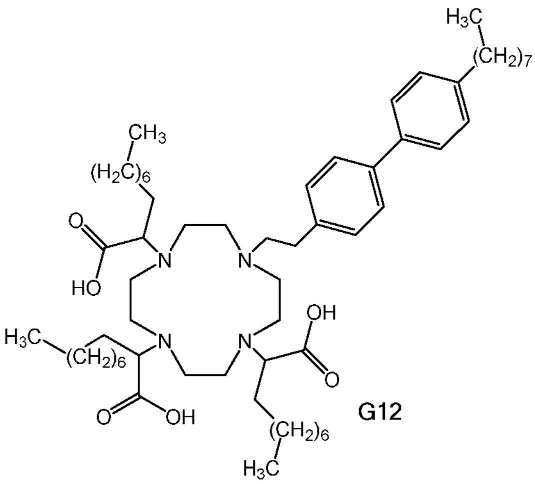

G12

ou leurs sels pharmaceutiquement acceptables.

Selon un mode de réalisation, les radicaux $R_3$, $R_4$ et $R_5$, de préférence $R_4$ sont choisis parmi :

- H, tert-butyle, phényle, $-CH_2-CH_2-$phényle, $-(CH_2)_7-CH_3$,
- 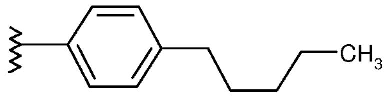,
- 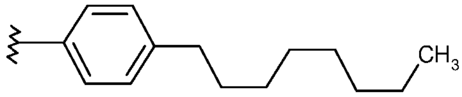,
- 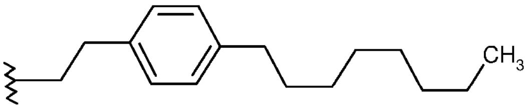,
- 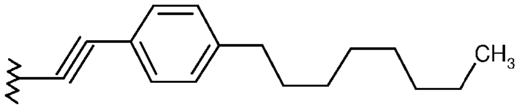,
- 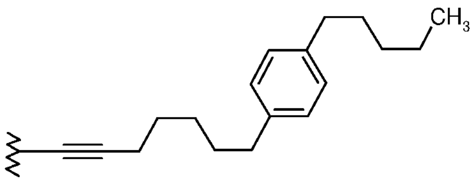,
- 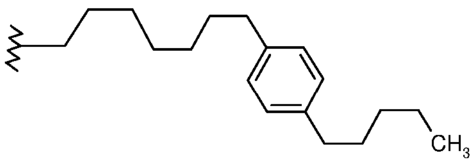,
- 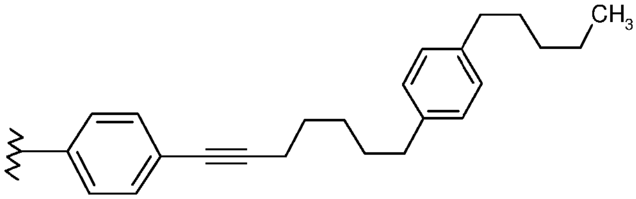,
- 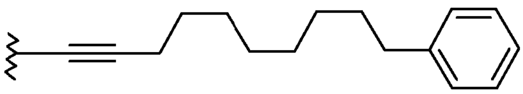,
- 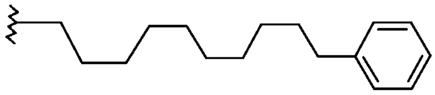,

- 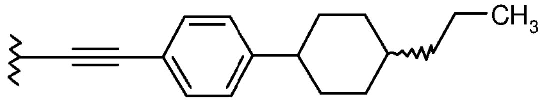
,

- 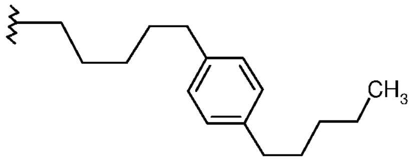
,

- 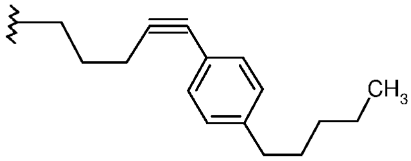
,

- 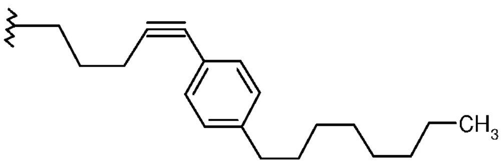
,

et

- 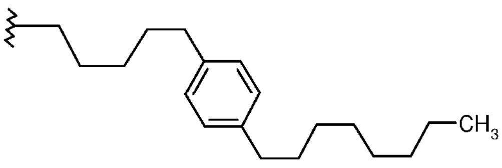
.

**Complexes**

L'invention concerne également un complexe d'un composé de formule (I) ou d'un de ses sels, tel que défini ci-dessus, avec un élément chimique M, de préférence un métal. Préférentiellement, M est un radioélément.

Selon un mode de réalisation, l'élément chimique M est un cation métallique choisi parmi le groupe constitué du bismuth (III), plomb (II), cuivre (II), cuivre (I), gallium (III), zirconium (IV), technétium (III), indium (III), rhénium (VI), astate (III), samarium (III), actinium (III), lutétium (III), terbium (III), holmium (III), gadolinium (III), europium (III) et yttrium (III), de préférence l'yttrium (III).

Selon un mode de réalisation particulier, l'élément chimique M est un radioélément choisi parmi le groupe constitué de $^{212}$Bi($^{212}$Pb), $^{213}$Bi(III), $^{64}$Cu(II), $^{67}$Cu(II), $^{68}$Ga(III), $^{89}$Zr(IV),$^{99m}$Tc(III), $^{111}$In(III), $^{186}$Re(VI), $^{188}$Re(VI),$^{211}$At(III), $^{225}$Ac(III), $^{153}$Sm(III), $^{149}$Tb(III), $^{166}$Ho(III), $^{212}$Pb(II), $^{177}$Lu(III) et $^{90}$Y(III), de préférence $^{212}$Bi($^{212}$Pb), $^{213}$Bi(III), $^{64}$Cu(II), $^{67}$Cu(II), $^{68}$Ga(III), $^{89}$Zr(IV),$^{99m}$Tc(III), $^{111}$In(III), $^{186}$Re(VI), $^{188}$Re(VI),$^{211}$At(III), $^{225}$Ac(III), $^{153}$Sm(III), $^{149}$Tb(III) et $^{166}$Ho(III), et encore plus préférentiellement $^{177}$Lu(III), $^{90}$Y(III) et $^{166}$Ho(III).

Selon un mode de réalisation, l'élément chimique M est choisi parmi Sc, Y, les lanthanides ou l'un de leurs isotopes radioactifs. De préférence, M est un radioélément choisi parmi les isotopes radioactifs de l'yttrium et des lanthanides. Selon un mode de réalisation, M est choisi parmi les lanthanides, les terres rares ou l'yttrium, ou l'un de leurs isotopes radioactifs.

Selon un mode de réalisation, M est choisi parmi : Sc, Y, La, Ce, Pr, Nd, Pm, Sm, Eu, Gd, Tb, Dy, Ho, Er, Tm, Yb et Lu.

Selon un mode de réalisation, M est choisi parmi les isotopes radioactifs suivants : $^{48}$Sc, $^{86}$Y, $^{90}$Y, $^{140}$La, $^{143}$Ce, $^{153}$Sm, $^{149}$Tb, $^{152}$Tb, $^{155}$Tb, $^{161}$Tb, $^{166}$Ho et $^{177}$Lu.

En particulier, parmi les radioéléments selon l'invention, on peut citer : $^{166}$Ho, $^{177}$Lu, $^{149}$Tb, $^{152}$Tb, $^{155}$Tb, $^{161}$Tb, $^{86}$Y, $^{90}$Y et $^{153}$Sm. Selon un mode de réalisation particulier, M est un radioélément choisi parmi le groupe constitué de

$^{166}Ho$, $^{177}Lu$ et $^{90}Y$.

Selon un mode de réalisation, ledit complexe est de formule générale (III) suivante :

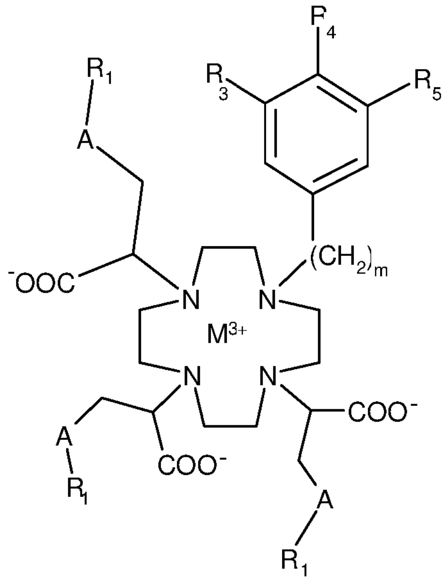

(III),

dans laquelle $R_1$, $R_3$, $R_4$, $R_5$, A, m et M sont tels que définis ci-dessus. En particulier, dans la formule générale (III), les groupements $COO^-$, permettant la complexation à l'élément M.

Selon un mode de réalisation, le rendement de radiomarquage des ligands selon l'invention est compris entre 10 et 100%, de préférence entre 75% et 99%.

**Procédé de préparation des composés de formule générale (I)**

L'invention concerne également un procédé de préparation des composés de formule générale (I), comprenant les étapes suivantes :

**a)** une étape d'alkylation du cyclène-glyoxal de formule (A) suivante :

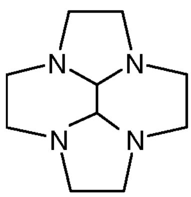

(A)

par un agent alkylant de formule (B1) suivante :

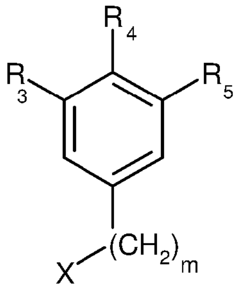

(B1)

avec $R_3$, $R_4$, $R_5$ et m étant tels que définis ci-dessus et X étant un halogène ou un groupe partant choisi parmi le

mésyle ($CH_3$-$SO_2$-O-), le tosyle ($CH_3$-Ph-$SO_2$-O-) et le triflyle ($CF_3$-$SO_2$-O-) ;
pour obtenir un composé de formule (IC) :

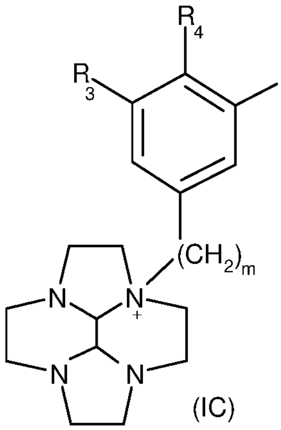

(IC)

dans laquelle $R_3$, $R_4$, $R_5$ et m sont tels que définis ci-dessus ;

**b)** une étape de déprotection du composé de formule (IC) pour obtenir un composé de formule (ID) suivante :

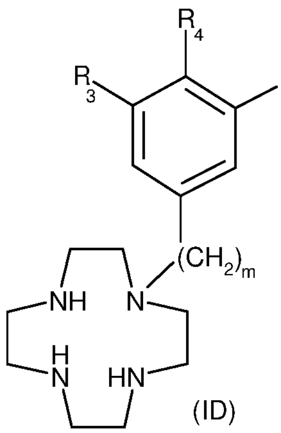

(ID)

dans laquelle $R_3$, $R_4$, $R_5$ et m sont tels que définis ci-dessus ;

**c)** une étape d'alkylation du composé de formule (ID) par un agent alkylant choisi parmi les composés de formule suivante (B2) :

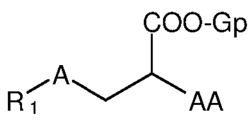

(B2)

avec AA étant choisi parmi les atomes d'halogène, le mésyle ($CH_3$-$SO_2$-O-) et le triflyle ($CF_3$-$SO_2$-O-) et dans laquelle $R_1$ et A sont tels que définis ci-dessus et Gp est un groupement protecteur de la fonction acide carboxylique, par exemple un ($C_1$-$C_5$)alkyle, de préférence un éthyle ;
pour obtenir un composé de formule (IF) suivante :

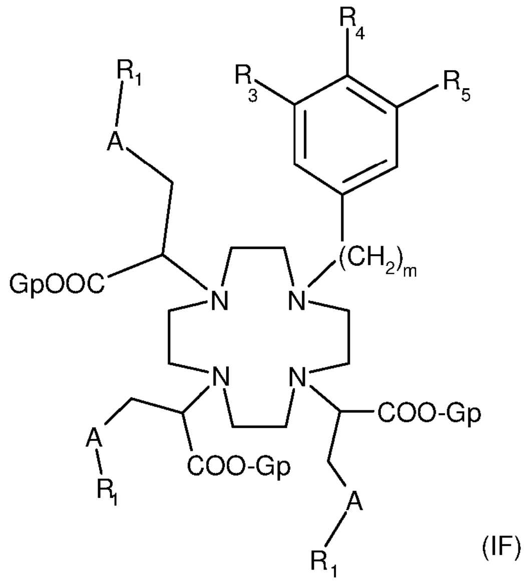

(IF)

dans laquelle Gp, A, $R_1$, $R_3$, $R_4$, $R_5$ et m sont tels que définis ci-dessus ;

**d)** une étape de saponification pour obtenir un composé de formule (I) tel que défini ci-dessus.

Selon un mode de réalisation, l'étape a) est réalisée en présence de toluène, à une température comprise entre 50°C et 70°C, par exemple environ 60°C. Selon un mode de réalisation, l'étape b) est réalisée en présence de potasse aqueuse ou d'hydrazine monohydratée. Selon un mode de réalisation, l'étape c) est réalisée en présence d'acétonitrile anhydre et de $K_2CO_3$. Selon un mode de réalisation, l'étape d) est réalisée en présence de KOH et d'éthanol. De préférence, les étapes a), b) et c) sont réalisées sous argon.

**Composition pharmaceutique**

L'invention concerne également une composition pharmaceutique comprenant un composé de formule (I) tel que défini ci-dessus ou un complexe tel que défini ci-dessus, et éventuellement un ou plusieurs excipient(s) pharmaceutiquement acceptable(s).

La composition peut en outre comprendre un tampon choisi parmi les tampons d'usage établi comme par exemple les tampons lactate, tartrate malate, maléate, succinate, ascorbate, carbonate, Tris((hydroxyméthyl)aminométhane), HEPES (acide 2-[4-(2-hydroxyéthyl)-1-pipérazine]éthanesulfonique), MES (acide 2-morpholino éthanesulfonique) et les mélanges de ceux-ci.

La composition pharmaceutique peut comprendre une phase huileuse, notamment une huile iodée telle que définie ci-dessus. Selon un mode de réalisation particulier, la composition pharmaceutique comprend en outre des esters éthyliques d'acides gras iodés de l'huile d'œillette.

Selon un mode de réalisation, la composition pharmaceutique selon l'invention est constituée d'une huile iodée et de complexes selon l'invention. Typiquement, la composition pharmaceutique selon l'invention est constituée de LIPIODOL® et de complexes selon l'invention. LIPIODOL® est constitué d'esters éthyliques d'acides gras iodés de l'huile d'œillette.

Selon un mode de réalisation, le taux d'extraction des complexes selon l'invention dans une phase huileuse telle que définie ci-dessus est compris entre 35% et 100%, de préférence entre 75% et 100%.

Les complexes selon l'invention sont notamment extractibles dans une phase huileuse. Ils permettent également d'obtenir des compositions comprenant une phase huileuse stable : en présence d'un milieu aqueux, tel qu'un sérum physiologique, la fuite dans le milieu aqueux des complexes selon l'invention est faible, par exemple comprise entre 0% et 20%, et ce jusqu'à au moins 15 jours.

De préférence, la composition pharmaceutique selon l'invention est radio-opaque, et donc visible par radiographie aux rayons X.

Selon un mode de réalisation particulier, la composition pharmaceutique est une composition injectable. Selon un mode de réalisation, la composition pharmaceutique selon l'invention est administrée par injection hépatique intra-artérielle.

L'invention concerne un complexe ou une composition pharmaceutique tels que définis ci-dessus, pour son

utilisation dans le traitement de cancers.

L'invention concerne également un complexe ou une composition pharmaceutique tels que définis ci-dessus, pour son utilisation en imagerie médicale.

L'invention concerne l'utilisation d'un complexe tel que défini ci-dessus pour la préparation d'un médicament pour le traitement de cancers.

L'invention concerne également l'utilisation d'un complexe ou d'une composition pharmaceutique tels que définis ci-dessus en imagerie médicale.

L'invention concerne une méthode de traitement thérapeutique d'un patient atteint d'un cancer, comprenant l'administration audit patient d'un complexe ou d'une composition pharmaceutique tels que définis ci-dessus. En particulier ladite méthode de traitement ne comprend pas d'étape de traitement chirurgical.

L'invention concerne également une méthode d'imagerie médicale d'une tumeur comprenant :

- une étape d'administration à un patient atteint d'un cancer d'un complexe ou d'une composition pharmaceutique selon l'invention ; et
- une étape de détection de la tumeur par une méthode d'imagerie médicale.

Par « cancer », on entend une prolifération cellulaire anormale (également appelée tumeur) au sein d'un tissu normal de l'organisme. Ces cellules cancéreuses dérivent toutes d'un même clone, cellule initiatrice du cancer qui a acquis certaines caractéristiques lui permettant de se diviser indéfiniment. Au cours de l'évolution de la tumeur, certaines cellules cancéreuses peuvent migrer hors de leur lieu de production et former des métastases.

Parmi les cancers, on peut notamment citer les cancers du foie, en particulier les cancers primitifs du foie, de préférence les hépatocarcinomes. Selon un mode de réalisation particulier, on peut citer parmi les cancers, l'hépatocarcinome, l'hémangioendothéliome épithélioïde, le cholangiocarcinome, les tumeurs neuro-endocrines et les métastases d'autres cancers telles que les métastases du cancer colorectal.

Selon un mode de réalisation particulier, le cancer est un carcinome hépatocellulaire au stade intermédiaire, chez l'adulte.

**Description des figures**

Figure 1 : La Figure 1 représente le rendement de radiomarquage en pourcentage pour des ligands selon l'invention.

Figure 2 : La Figure 2 représente le taux d'extraction dans la phase huileuse en pourcentage pour des complexes selon l'invention.

Figure 3 : La Figure 3 représente le test de stabilité dans le sérum physiologique en donnant le taux de fuite en pourcentage en fonction des radiotraceurs selon l'invention.

Figure 4 : La Figure 4 représente le taux de fuite en pourcentage dans le sérum physiologique des radiotraceurs H5 (a) et H6 (b) selon l'invention.

Figure 5 : La Figure 5 représente le taux de fuite en pourcentage dans le sérum physiologique des radiotraceurs H5 (a) et H6 (b) selon l'invention.

**EXEMPLES**

**I. Conditions expérimentales générales** :

Les produits commerciaux ainsi que les solvants utilisés pour ces synthèses proviennent essentiellement des sociétés Sigma-Aldrich®, Merck®, Interchim® et VWR®.

La température ambiante de la pièce varie, en général, entre 20°C et 25°C. Les évaporations des solvants sont réalisées sous pression réduite, à l'aide d'un évaporateur Buchi R-210, à des températures d'environ 40°C. Le suivi des réactions et des purifications se fait par chromatographie sur couche mince (CCM) en utilisant des plaques de verre de silice (silica gel 60 F254), révélées sous UV et par l'iode. Les purifications sont réalisées sur des appareils de chromatographie flash GRACE Reveleris® ou CombiFlash® Rf provenant de Teledyne Isco®. Les cartouches utilisées pour les purifications sur silice sont essentiellement des cartouches GRACE Reveleris® (40 μm, 4 ou 12g). Les réactions activées avec les micro-ondes ont été réalisées dans le réacteur Monowave Series Anton Paar®.

Les purifications par HPLC préparative sont réalisées sur PuriFlash® 4250 avec colonne Symmetry (150x30mm ; 5μm).

Les chromatogrammes HPLC sont enregistrés sur un appareil Agilent Technologies® de la série 1200. La détection se fait généralement à 201 nm et 270 nm. Dans certains cas, un détecteur Corona ou ELSD est nécessaire.

Les colonnes utilisées proviennent de différents fournisseurs : Waters® (Symmetry C18), Phenomenex® (Luna C8) et ACE® (C4 ACE). Les analyses de spectrométrie de masse ont été réalisées par chromatographie liquide couplée à un spectromètre de masse amaZon X de Bruker®.

**II. Synthèse des ligands** :

*Note : dans les exemples suivants, le terme « Ar » désigne un phényle substitué par les radicaux $R_3$, $R_4$ et $R_5$ tel que selon l'invention.*

**Exemple 1 : Préparation d'agents alkylants**

1- Procédure générale pour transformation des dérivés alcools

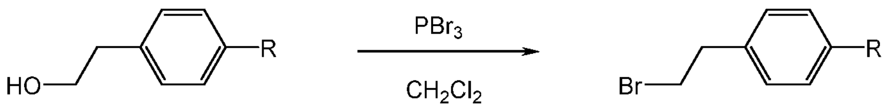


Bromation :

Le dérivé alcool est dilué dans le dichlorométhane puis le $PBr_3$ (solubilisé dans le dichlorométhane) est ajouté goutte à goutte à la solution préalablement refroidie dans un bain de glace/acétone. Le milieu réactionnel est agité à température ambiante pendant 2 heures puis traité avec 10mL d'eau. La phase organique est récupérée, purifiée sur bouchon de silice (Heptane/DCM (1 :1)), séchée sur $Na_2SO_4$ et concentrée à sec.

Mésylation :

Dans un tricol est ajouté l'alcool (3,64mmol, 1 équivalent) dilué dans 15mL de DCM (4mL/mmol). La solution est refroidie dans un bain eau/glace et à travers un septum $Et_3N$ (2 équiv) et le chlorure de mésyle (1,2 équivalents) sont ajoutés gouttes à gouttes. Le milieu réactionnel est agité pendant 10 minutes puis 15mL d'eau sont ajoutés. La phase organique est récupérée, séchée sur $Na_2SO_4$ et concentrée à sec. Obtention d'un solide jaune qui sera purifié sur bouchon de silice avec le mélange Heptane/DCM (4 :6).

Tableau 1 : Préparation de certains agents alkylants B

| ***Agent alkylant B*** | ***Produit attendu*** | ***Type*** | ***Rendement*** | ***Masse moléculaire (g/mol)*** |
|---|---|---|---|---|
| 1-bromo-8-phenyloctane | 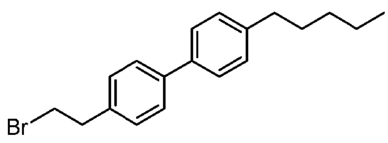 | Bromation | 31% | 331.30 |
| 4-(2-Bromoethyl)-4'-octyl-1,1'-biphenyl | 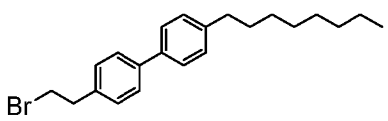 | Bromation | 33.3% | 372.15 |
| 4-(2-Bromoethyl)-4'-pentyl-1,1'-biphenyl | 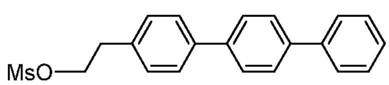 | Mésylation | 43% | 352.11 |

**Exemple 2 : Alkylation du cyclène-Glyoxal A**

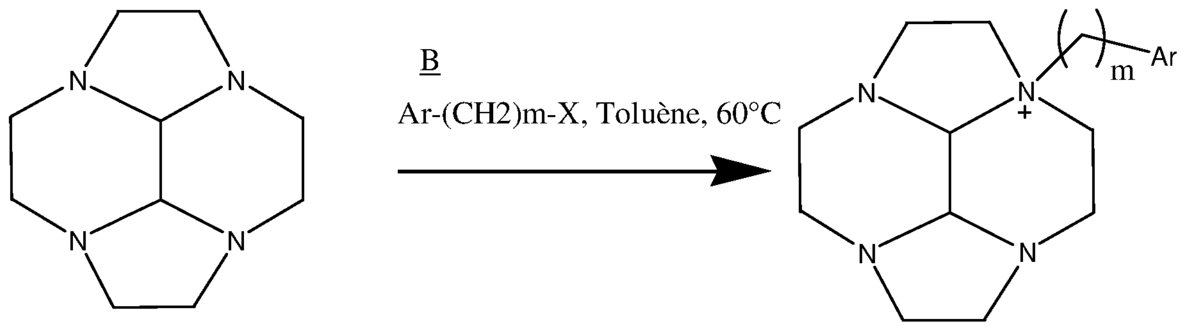


A C

Le cyclène glyoxal A (1,2éq, 5,6 mmole) est dissout dans 4mL de toluène et l'agent alkylant B avec X est un halogène ou un mésyle (1éq, 2,16mmole) est ajouté. Le milieu réactionnel est ensuite agité sous argon à 60°C pendant 5 heures à 5 jours (suivant la nature de l'agent alkylant B). A la fin de la réaction, le solide qui a précipité est filtré et lavé abondamment au toluène. Après séchage au dessiccateur le produit C obtenu est utilisé sans autre purification.

*Tableau 2 : alkylation du cyclène glyoxal A*

| ***Agent alkylant B*** | ***Produit attendu C*** | ***Temps de réaction*** | ***Rdt*** | ***M******* | ***MS (ES+)****** | ***HPLC (temps de rétention (tr)*** |
|---|---|---|---|---|---|---|
| (Bromométhyl)-1-di-tert-butyl-3,5-benzène | 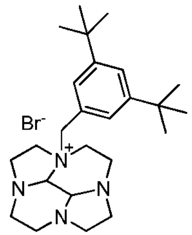 | 5 heures | 97% | 374,6 | $C_{23}H_{42}N_4$ 397,2 $[M+Na]^+$ | 6,16 min* |
| (Bromométhyl)-1-di(trifluorométhyl)-3,5-benzène | 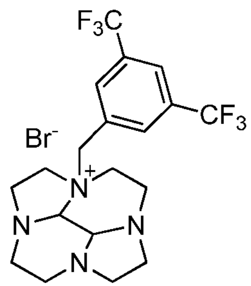 | 4 jours | 90% | 421,2 | $C_{19}H_{23}F_6N_4Br$ 421,1 | 5,23 min* |
| Chlorométhyl-1-n-octyl-4-benzène | 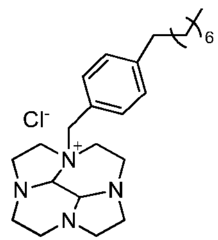 | 18 heures | 57% | 397,3 | $C_{25}H_{41}N_4$ 397,2 | 5,4 min* |
| (Chlorométhyl)-4-diphenyl-1,2-ethane | 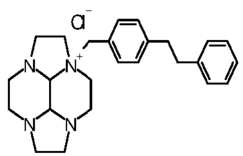 | 24 heures | 74% | 389.6 | $C_{25}H_{33}N_4$ 389.5 | 5.8 min* |

(suite)

| ***Agent alkylant B*** | ***Produit attendu C*** | ***Temps de réaction*** | ***Rdt*** | ***M******* | ***MS (ES+)****** | ***HPLC (temps de rétention (tr)*** |
|---|---|---|---|---|---|---|
| Chloro-4-Phénylbenzyle | 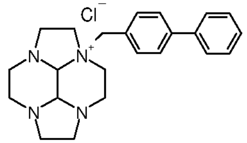 | 3 jours | 80% | 361.5 | $C_{23}H_{29}N_4$ 361.1 | 7.39 min* |
| (Bromo-2-éthyl)-méthyl-3-benzene | 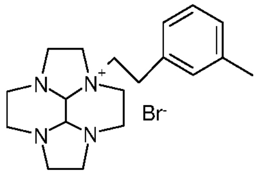 | 48 heures | 70-90% | 313.47 | $C_{19}H_{29}N_4$ 313 | 4.77 min* |
| (Bromo-3-propyl)-benzène | 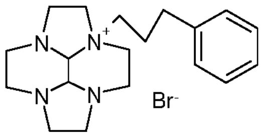 | 48 heures | 98-% | 313.47 | $C_{19}H_{29}N_4$ 313 | 4.64 min* |
| 1-bromo-8-phenyloclane | 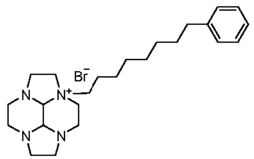 | 60 heures | 79% | 383.6 | $C_{24}H_{39}N_4$ 383.35 $[M+H]^+$ | 6.96 min** |
| 4-(2-Bromoethyl)-4'-octyl-1,1'-biphenyl | 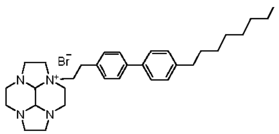 | 48 heures | 56% | 487.76 | $C_{32}H_{47}N_4$ | 9.36 min** |
| 4-(2-Bromoethyl)-4'-pentyl-1,1'-biphenyl | 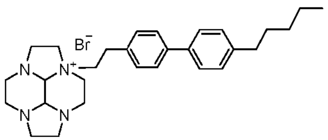 | 60 heures | 76% | 445.68 | $C_{29}H_{41}N_4$ 445.38 $[M+H]^+$ | 8.1 min** |

(suite)

| *Agent alkylant B* | *Produit attendu C* | *Temps de réaction* | *Rdt* | *M*** ** | *MS (ES+)**** | *HPLC (temps de rétention (tr)* |
|---|---|---|---|---|---|---|
| 4-(2-mesylethyl)-1,1':3',1"-terphenyl | 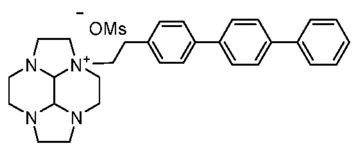 | 24 heures | 90% | 466.66 | $C_{30}H_{35}N_4$ | 10.65 min** |

*Colonne Sunfire™ C-18 (Waters®), 3.5µm, 150x4.6 mm, 98/2 Eau (0.05% HCOOH)/ $CH_3CN$ en 10 min 100% $CH_3CN$
**Colonne Symmetry C-18, Waters, 3.5µm, 150x4.6 mm; 98/2 Eau (0.05% TFA)/ $CH_3CN$ en 12min 100% $CH_3CN$ et 8 min à 100%.
*** MS (ES+) correspond aux résultats de spectrométrie de masse avec une modalité d'ionisation electrospray en mode positif.
M**** : Masse moléculaire (g/mole)

**Exemple 3 : Déprotection du cyclène-glyoxal monoalkylé C**

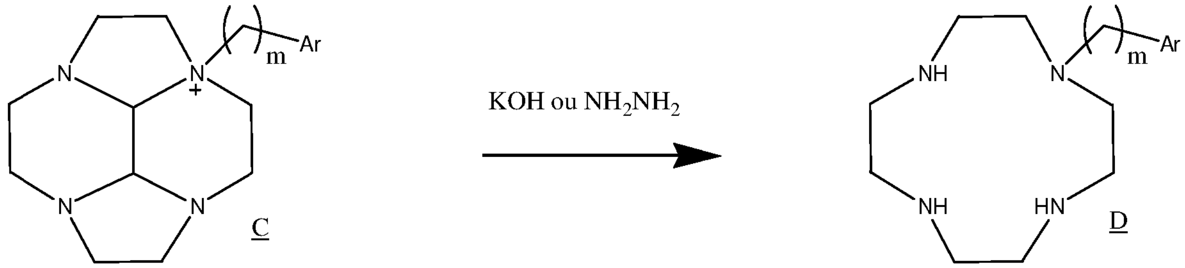


Le cyclène glyoxal monoalkylé C (1éq, 1,5mmole) est dissout dans de la potasse aqueuse (20%, 10mL) ou dans de l'hydrazine monohydraté ($NH_2$-$NH_2$-$H_2O$, 3mL). Le milieu réactionnel est ensuite chauffé et agité sous argon.

- *Avec chauffage micro-ondes* : Le produit est introduit dans un tube en verre avec la solution de potasse 2M et celui-ci est placé dans la cavité du réacteur Anton Paar. Le cycle de chauffage est réalisé en programmant une rampe de température de 10 minutes puis un chauffage à température constante (180°C) durant 1 heure.

Traitement :

- *Réaction avec la potasse* : Le milieu est extrait au chloroforme (x3). Les phases organiques sont réunies, séchées, filtrées puis évaporées. Le produit souhaité D est obtenu sous forme d'huile ou de solide. Dans certains cas, une purification sur colonne d'alumine basique est nécessaire.
- *Réaction avec l'hydrazine* : Le milieu réactionnel est refroidi. Le solide obtenu est alors filtré puis repris dans l'éthanol. Le milieu est concentré sous pression réduite. Le produit désiré D est obtenu sous forme d'huile ou de solide. Dans certains cas, une purification sur colonne d'alumine basique est nécessaire.

*Tableau 3 : Déprotection du cyclène-glyoxal monoalkylé C*

| ***Produit D*** | **Base** | ***Temps de réaction*** | ***Rdt*** | ***M******* | ***MS (ES+)*** | ***HPLC (temps de rétention (tr)*** |
|---|---|---|---|---|---|---|
| 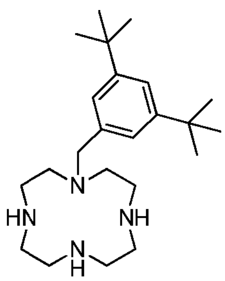 | Potasse | 5 jours | 81% | 374,6 | $C_{23}H_{42}N_4$ 397,25 $[M+Na]^+$ | 3,3 min* |
| | Hydrazine | 4 heures | | | | |
| 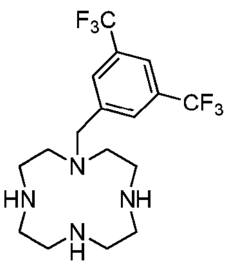 | Potasse | 5 jours | 84% | 398,4 | $C_{17}H_{24}F_6N_4$ 421,10 $[M+Na]^+$ | 1,8 min* |
| | Hydrazine | 18 heures | | | | |

(suite)

| ***Produit D*** | **Base** | ***Temps de réaction*** | ***Rdt*** | ***M******* | ***MS (ES+)*** | ***HPLC (temps de rétention (tr)*** |
|---|---|---|---|---|---|---|
| 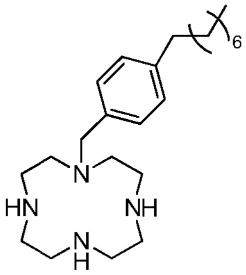 | Hydrazine | 18 heures puis 3 jours à 20 °C | 32% | 374,6 | $C_{23}H_{42}N_4$ 375,25 $[M+H]^+$ | 3,5 min* |
| 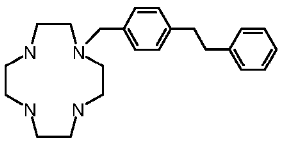 | Hydrazine | 18 heures | 91% | 366.6 | $C_{23}H_{34}N_4$ 367,19 $[M+H]^+$ | 1.8 min* |
| 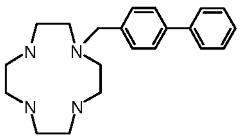 | Potasse | 6 jours à 60°C | 83% | 338.5 | $C_{21}H_{30}N_4$ 339.16 [M+H] + | 7.63 min* |
| 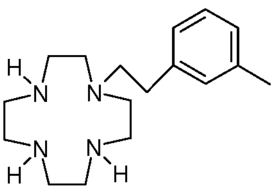 | Hydrazine | 2 heures 100°c | 92% | 290.46 | C17H30N4 291$[M+H]^+$ | 4.59 min* |
| 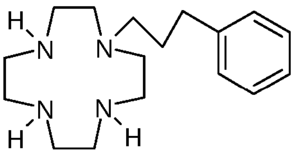 | Hydrazine | 2 heures 100° | 90% | 290.46 | C17H30N4 291$[M+H]^+$ | 4.84 min* |
| 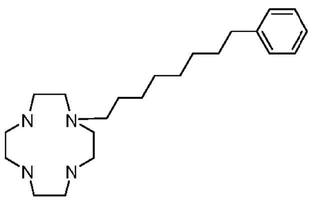 | Hydrazine | 2 heures | 76% | 360.59 | C22H40N4 361.25 $[M+H]^+$ | 7.12 min** |
| 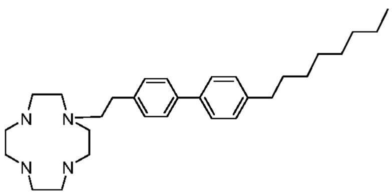 | Hydrazine | 4 heures | 85% | 464.74 | $C_{30}H_{48}N_4$ | 9.0 min** |

(suite)

| *Produit* *D* | **Base** | ***Temps de réaction*** | ***Rdt*** | ***M******* | ***MS (ES+)*** | ***HPLC (temps de rétention (tr)*** |
|---|---|---|---|---|---|---|
| 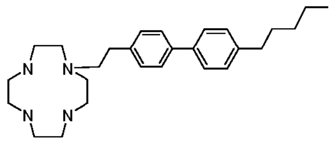 | Hydrazine | 2 heures | 56% | 422.66 | $C_{27}H_{42}N_4$ 423.4 $[M+H]^+$ | 8.03 min** |
| *Colonne Sunfire™ C-18 (Waters®), 3.5μm, 150x4.6 mm, 98/2 Eau (0.05% HCOOH)/ $CH_3CN$ en 10min 100% $CH_3CN$ <br> **Colonne Symmetry C-18, Waters, 3.5μm, 150x4.6 mm 98/2 Eau (0.05% TFA)/ CH3CN en 12min 100% $CH_3CN$ et 8 min à 100% <br> M****: Masse moléculaire (g/mole) | | | | | | |

**Exemple 4 : Alkylation du cyclène monoalkylé D**

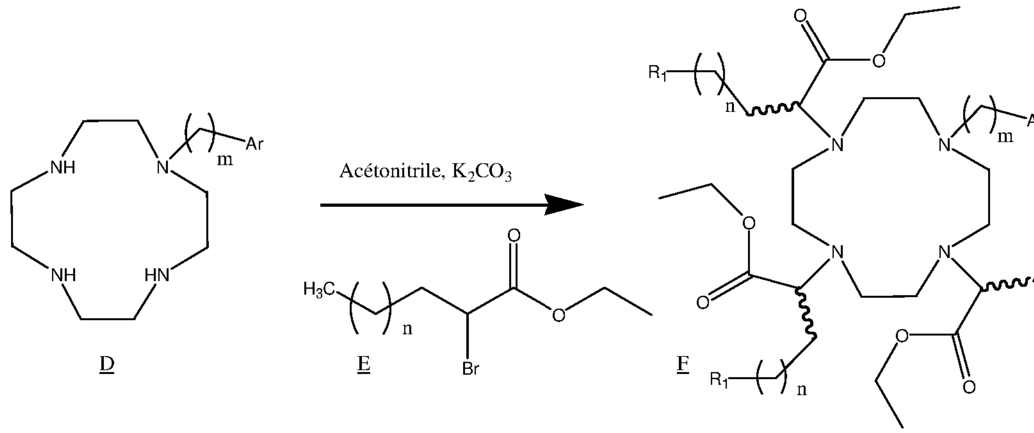


D E F

*Tableau 4 : Agents alkylants E*

| **Structures de E** | **Noms** | **Formules brutes, RN, PM (g/mole)** |
|---|---|---|
| 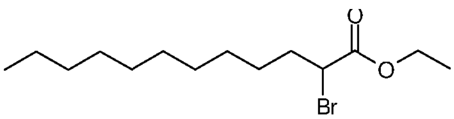 | Ester éthylique de l'acide Bromo-2-Dodécanoique racémique E1 | $C_{14}H_{27}BrO_2$, 6974-87-4, 307.27 |
| 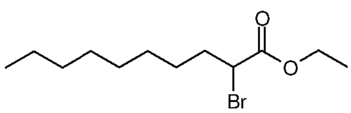 | Ester éthylique de l'acide Bromo-2-Décanoique racémique E2 | $C_{12}H_{23}BrO_2$, 6974-85-2, 279.22 |
| 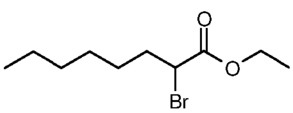 | Ester éthylique de l'acide Bromo-2-Octanoique racémique E3 | $C_{10}H_{19}BrO_2$ 5445-29-4 251.16 |

Le cyclène mono-alkylé D (1éq, 1,5mmole) est dissout dans l'acétonitrile anhydre (5mL) et $K_2CO_3$ (3,2éq, 4,8mmole) est ajouté. L'agent alkylant E (3,5éq, 5,25mmole), préalablement dissout dans l'acétonitrile (3mL), est ajouté goutte à goutte au mélange. Le milieu réactionnel est ensuite chauffé à reflux pendant 18 heures sous argon. A la fin de la réaction, les sels sont filtrés et le filtrat évaporé. L'huile obtenue contenant F est ensuite purifiée par chromatographie flash sur colonne de silice : 100% de dichlorométhane puis ajout progressif de méthanol jusqu'à la proportion 80/20.

*Tableau 5 : Alkylation du cyclène monoalkylé D*

| Types | Codes | Substituants | Rendements | Masses moléculaires (g/mole) | MS (ES+) | HPLC (temps de rétention (tr) |
|---|---|---|---|---|---|---|
| C12 | F1 | -N-(3,5-di-tert-butyl) benzyle | 31% | 1053.70 | $C_{65}H_{120}N_4O_6$ | - |
| | F2 | -N-(4-n-octyl)benzyle | 29% | 1053.70 | $C_{65}H_{120}N_4O_6$ 1053.9 $[M+H]^+$ | 10.6 min* |
| C10 | F3 | -N-(3,5-di-tert-butyl) benzyle | 46% | 969,5 | $C_{59}H_{108}N_4O_6$ 969,98 $[M+H]^+$ | 9,9 min* |
| | F4 | -N-[3,5-Bis (trifluométhyl]benzyle | 36% | 992,7 | $C_{53}H_{90}F_6N_4O_6$ 993,71 $[M+H]^+$ | 9,9 min* |
| | F5 | N-[4-(2-PhenylEthyl)-benzyl] | 23% | 961.48 | $C_{59}H_{100}N_4O_6$ 961.92 | 8.9 min* |
| | F6 | -N-(4-phenyl)benzyle | 17.5% | 933.46 | $C_{57}H_{96}N_4O_6$ 933.78 | 8.5 min* |
| | F7 | N-(4-n-octyl)benzyle | 55% | 969.54 | $C_{59}H_{108}N_4O_6$ | 9.6 min* |
| | F11 | **N-(2-(4'-pentyl-[1,1'-biphenyl]-4-yl) ethyl)** | 21% | 1017.58 | $C_{63}H_{108}N_4O_6$ 1017.90 $[M+H]^+$ | 10.9 min** |
| | F12 | **N-(2-(4'-octyl-[1,1'-biphenyl]-4-yl) ethyl)** | 30% | 1059.66 | $C_{66}H_{114}N_4O_6$) 1017.85 $[M+H]^+$ (triester méthylique)*** | 11.23min, 11.81 min** |
| C8 | F8 | -N-(4-n-octyl)benzyle | 33% | 884,7 | $C_{52}H_{94}N_4O_6$ 885,85 $[M+H]^+$ | 8,5 min* |
| | F9 | -N-[4-(2-PhenylEthyl)-benzyl] | 56% | 877.3 | $C_{53}H_{88}N_4O_6$ 877.82 $[M+H]^+$ | 8.0 min* |
| *Colonne Sunfire™ C-18 (Waters®), 3.5µm, 150x4.6 mm, 98/2 Eau (0.05% HCOOH)/ $CH_3CN$ en 10min 100% $CH_3CN$<br>**Colonne Symmetry C-18, Waters, 3.5µm, 100x4.6 mm 98/2 Eau (0.05% TFA)/ $CH_3CN$ en 8min 100% $CH_3CN$ et 5min à 100%<br>***Transestérification en cours de réaction. | | | | | | |

**Exemple 5 : Alkylation du cyclène monoalkylé D, Alkylation stéréospécifique**

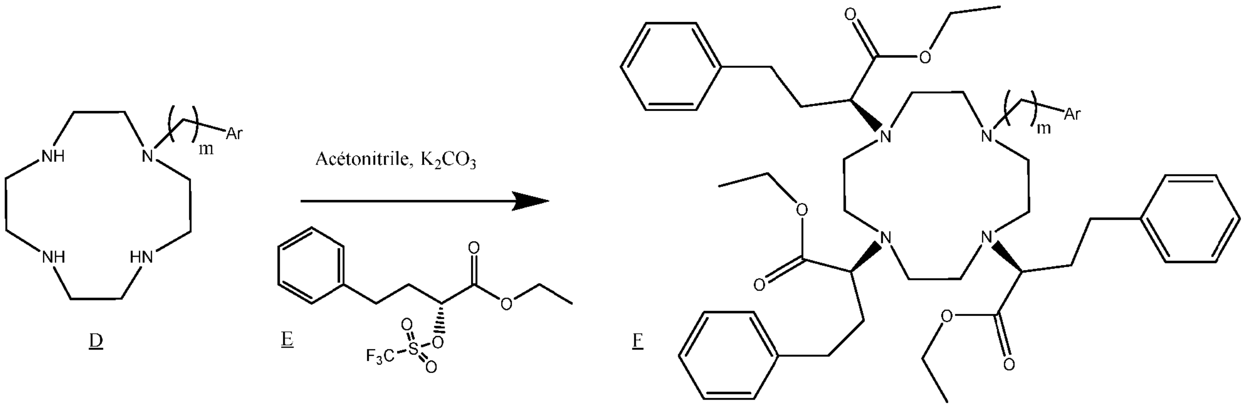


*Tableau 6 : Agent alkylant E*

| Structure de E | Nom | Formule brute, RN, PM (g/mole) |
|---|---|---|
| 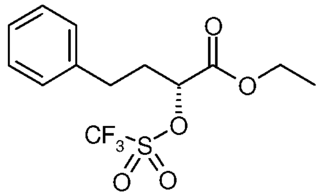 | Ester éthylique de l'acide Phényl-4-butanoique de configuration R E4 | $C_{13}H_{15}F_3O_5S$, 88767-98-0 340.32 |

-Procédure de préparation de l'agent alkylant triflate E4:

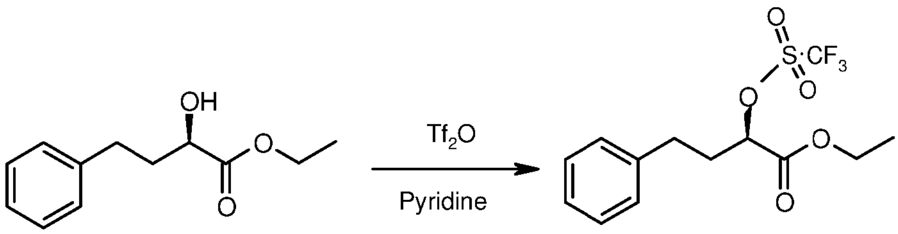


A une solution de 15 mmoles d'ester éthylique de l'acide R-(-) Hydroxy-2-phényl-4-butyrique dans 10 ml de $CH_2Cl_2$ et 1,2 ml de pyridine, est ajoutée goutte à goutte, à 0°C, une solution d'anhydride triflique dans 5ml de $CH_2Cl_2$, sous argon.

Le milieu obtenu est maintenu à cette même température pendant 1h puis 1h à 15°C et la nuit à température ambiante. Après filtration pour éliminer les sels de pyridinium, le filtrat est concentré puis chromatographié sur $SiO_2$ avec un éluant de composition cyclohexane 5 /AcOEt 5. Les fractions sélectionnées puis évaporées donnent une huile translucide avec un rendement de 53% qui sera engagé dans l'étape suivante, rapidement.
CCM : Rf = 0.5 avec éluant AcOEt 5/Cyclohexane 5.

-Procédure d'alkylation avec l'agent alkylant triflate E4:

A une solution de 3.8 mmoles de cyclène monofonctionnalisé D dans 10 ml de $CH_3CN$ et 0.54 ml de diisopropyléthylamine, est ajoutée, goutte à goutte, sous Argon et à température ambiante, une solution du réactif triflate E4 précédemment préparé (1.3 mmole dans 10 ml de $CH_3CN$). Après 18h de réaction à température ambiante, le milieu réactionnel est filtré puis concentré avant d'être chromatographié sur $SiO_2$ avec un éluant de composition $CH_2Cl_2$/MeOH. Les fractions mélangées et évaporées donnent une huile ambrée (rendement 41%).

*Tableau 7: Alkylation du cyclène monoalkylé D, Alkylation stéréospécifique*

| **Type** | ***Code*** | ***Substituant*** | ***Rendement*** | ***Masse moléculaire (g/mole)*** | ***MS (ES+)*** | ***HPLC (temps de rétention (tr)*** |
|---|---|---|---|---|---|---|
| **PheEth** | **F10** | **N-(4-phenyl) benzyle** | 41% | 909.23 | $C_{57}H_{72}N_4O_6$ 909.7<br>$[M+H]^+$ 455.38<br>$[M+2H]^{2+}$ | **Tr = 8.52** |
| *Colonne Sunfire™ C-18 (Waters®), 3.5μm, 150x4.6 mm | | | | | | |

**Exemple 6 : Procédure générale pour la saponification de F**

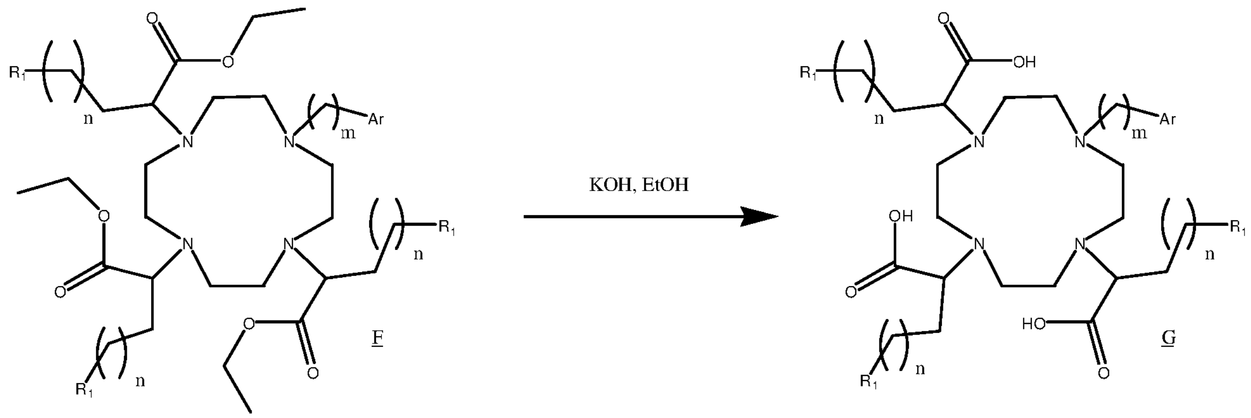


F G

Le ligand sous forme d'ester éthylique F (1éq, 0,5mmole) est dissout dans l'éthanol (5mL) et une solution de potasse alcoolique (2mole/L, 10mL) est ajoutée. Le milieu réactionnel est ensuite agité au reflux 18 heures. Après retour à température ambiante, l'éthanol est évaporé. L'ajout d'acide chlorhydrique (1mole/L) jusqu'à pH = 1 conduit à la précipitation d'un solide. Celui-ci est filtré et lavé abondamment à l'eau afin éliminer les sels et conduire au ligand G.

Pour le ligand G1, il a été possible, au stade ester de séparer quatre fractions lors de la purification par chromatographie sur silice. Ces quatre fractions ont été saponifiées séparément pour conduire à 4 fractions de ligand (G1-Iso1 à G1-Iso4).

*Tableau 8 : Saponification*

| ***Ligands G selon l'invention*** | | | | | | |
|---|---|---|---|---|---|---|
| **Types** | ***Codes*** | ***Produits*** | ***Rendements*** | ***Masses moléculaires (g/ mole)*** | ***MS (ES+)*** | ***HPLC (temps de rétention (tr)*** |
| **C12** | **G1** | **-N-(3,5-di-tert-butyl)benzyle** 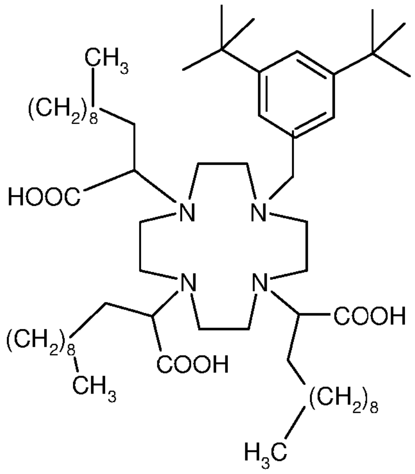 | 81% | 969.54 | $C_{59}H_{108}N_4O_6$ 969.8 $[M+H]^+$ | - |
| | **G2** | **-N-(4-n-octyl)benzyle** 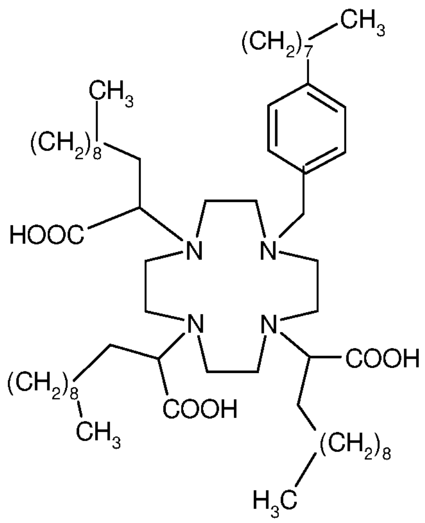 | 94% | 969.54 | $C_{59}H_{108}N_4\ O_6$ 969.8 $[M+H]^+$ | 9.9 min* |

(suite)

| *Ligands G selon l'invention* | | | | | | |
|---|---|---|---|---|---|---|
| **Types** | ***Codes*** | ***Produits*** | ***Rendements*** | ***Masses moléculaires (g/mole)*** | ***MS (ES+)*** | ***HPLC (temps de rétention (tr)*** |
| **C10** | **G3** | **-N-(3,5-di-tert-butyl)benzyle** 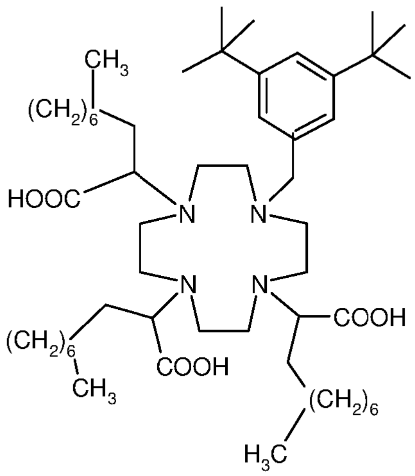 | 19% (pureté : 93%) | 884,7 | $C_{53}H_{96}N_4O_6$ 885,72 $[M+H]^+$ | - |
| | **G4** | **-N-[3,5-Bis(trifluométhyl]benzyle** 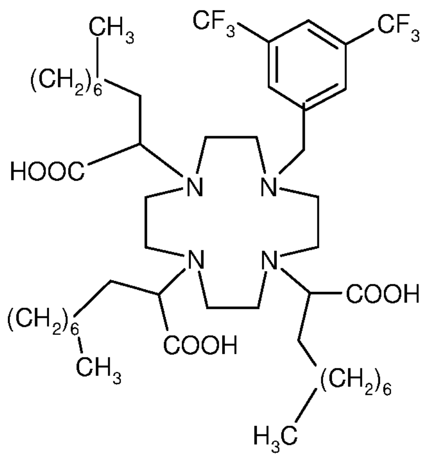 | 30% | 908,6 | $C_{47}H_{78}F_6N_4O_6$ 909,59 $[M+H]^+$ | - |

(suite)

| *Ligands G selon l'invention* | | | | | | |
|---|---|---|---|---|---|---|
| **Types** | ***Codes*** | ***Produits*** | ***Rendements*** | ***Masses moléculaires (g/mole)*** | ***MS (ES+)*** | ***HPLC (temps de rétention (tr)*** |
| | **G5** | **-N-[4-(2-PhenylEthyl)-benzyl]** 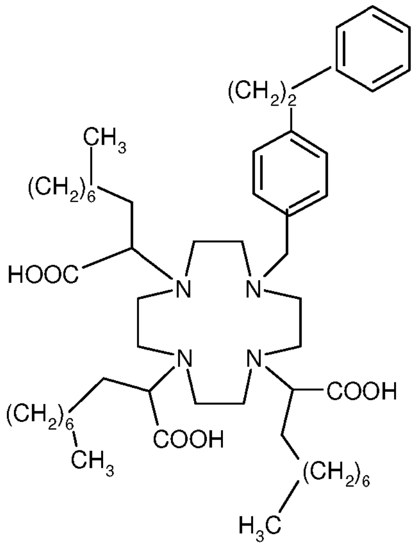 | 61% | 877.32 | $C_{53}H_{88}N_4O_6$ 877.73 $[M+H]^+$ | 7.8 min* |
| | **G6** | **-N-(4-phenyl)benzyle** 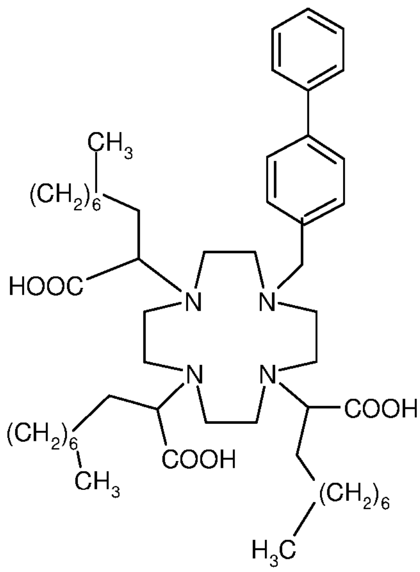 | 90% | 849.26 | $C_{51}H_{84}N_4O_6$ 849.72 [M+H°] | 7.5 min* |

(suite)

| *Ligands G selon l'invention* | | | | | | |
|---|---|---|---|---|---|---|
| **Types** | ***Codes*** | ***Produits*** | ***Rendements*** | ***Masses moléculaires (g/mole)*** | ***MS (ES+)*** | ***HPLC (temps de rétention (tr)*** |
| | **G7** | **-N-(4-n-octyl)benzyle** 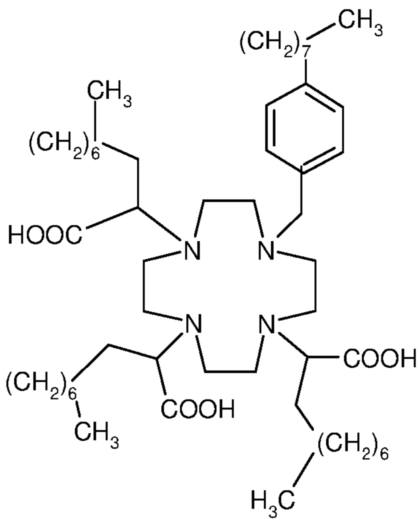 | 86% | 885.38 | $C_{53}H_{96}N_4O_6$ 885.7 $[M+H]^+$ | 8.4 min* |
| **C8** | **G8** | **N-(4-n-octyl)benzyle** 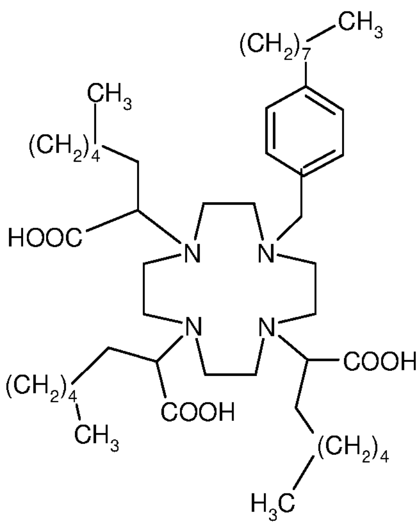 | 25% | 801,6 | $C_{47}H_{84}N_4O_6$ 801.6 $[M+H]^+$ | - |

(suite)

| *Ligands G selon l'invention* | | | | | | |
|---|---|---|---|---|---|---|
| **Types** | ***Codes*** | ***Produits*** | ***Rendements*** | ***Masses moléculaires (g/mole)*** | ***MS (ES+)*** | ***HPLC (temps de rétention (tr)*** |
| | **G9** | **-N-[4-(2-PhenylEthyl)-benzyl]** 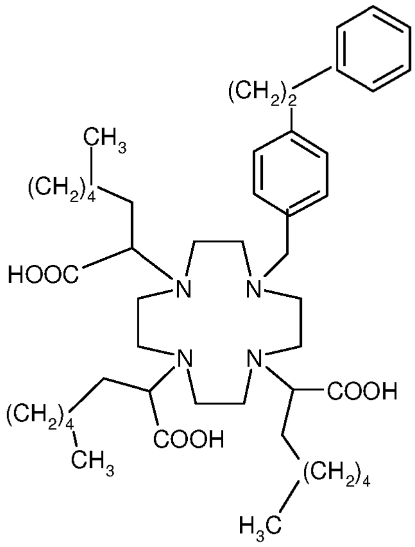 | 70% | 793.2 | $C_{47}H_{76}N_4O_6$ 793.59 $[M+H]^+$ | 6.4 min* |
| **PheEth.** | **G10** | **-N-(4-phenyl)benzyle** 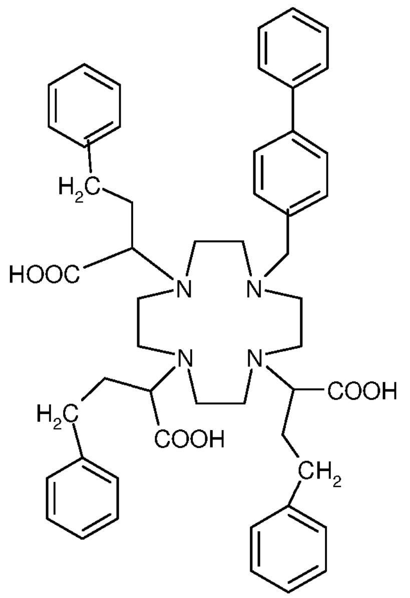 | 20% | 825,07 | 826 $[M+H]^+$ 413 $[M+2H]^{2+}$ | 8.08 min* |

(suite)

| Ligands G selon l'invention | | | | | | |
|---|---|---|---|---|---|---|
| **Types** | ***Codes*** | ***Produits*** | ***Rende ments*** | ***Masses molécul aires (g/ mole)*** | ***MS (ES+)*** | ***HPLC (temps de rétention (tr)*** |
| **C10** | **G11** | **N-(2-(4'-pentyl-[1,1'-biphenyl]-4-yl)ethyl)** 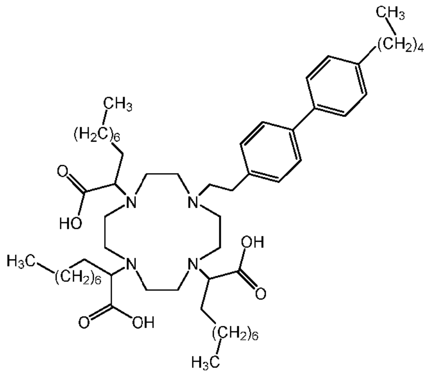 | 90% | 933.42 | C57H96N4O6 [M+H]+ 933.8 | 9.8 min 10.0 min** |
| | **G12** | **-(2-(4'-octyl-[1,1'-biphenyl]-4-yl)ethyl)** 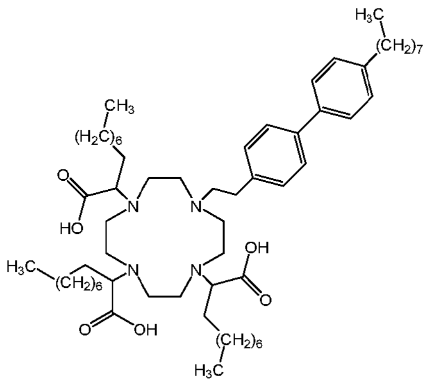 | 33% | 975.50 | C60H102N4O6 [M+H]+ 975.8 | 10.3 min** |
| *)Colonne Sunfire™ C-18 (waters®), 3.5µm, 150x4.6 mm,98/2 Eau (0.05% HCOOH)/ $CH_3CN$ en 10min 100% $CH_3CN$ **) Colonne Symmetry C-18, Waters, 3.5µm, 100x4.6 mm 98/2 Eau (0.05% HCOOH)/ $CH_3CN$ en 12min 100% $CH_3CN$ et 8 min à 100%* | | | | | | |

**III. Synthèse des complexes :**

**Exemple 7 : Procédure générale pour la complexation de G avec l'yttrium 89**

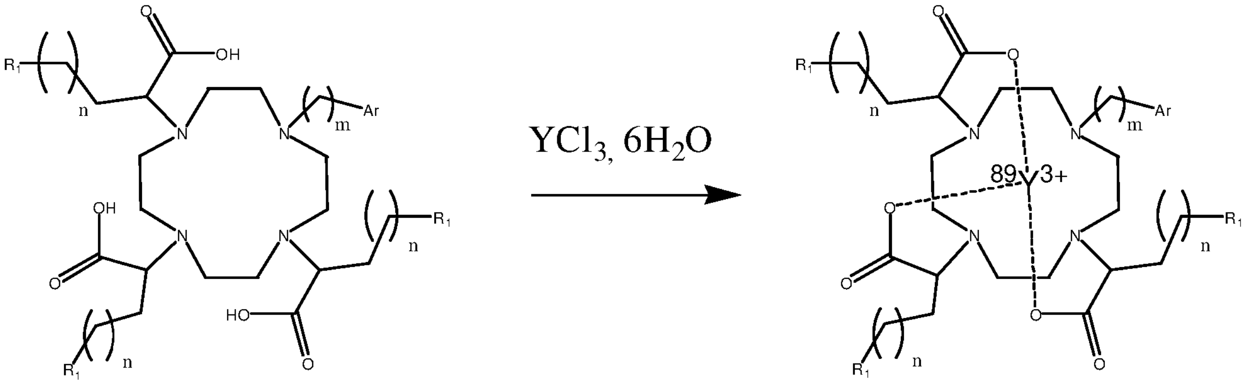


G H

Le ligand G (1éq, 0,07mmole) est dissout dans 3mL de méthanol (pH=6). Le chlorure d'yttrium hexahydraté (1,5éq, 0,1mmole) est ensuite ajouté (pH=4).Il est ensuite procédé à l'ajout contrôlé d'une solution de méthanolate de sodium pour obtenir un pH neutre. Le milieu est agité et chauffé à 65°C pendant une nuit. A la fin de la réaction, le solvant est évaporé et le solide obtenu est lavé abondamment à l'eau pour conduire au complexe H.

*Tableau 9 : Complexation de G avec l'yttrium 89*

| ***Complexes d'Yttrium 89 H*** | | | | | |
|---|---|---|---|---|---|
| **Types** | **Codes** | ***Produits*** | ***Rendements*** | ***Masses moléculaires (g/mole)*** | ***MS (ES+)*** |
| **C12** | **H2** | **-N-(4-n-octyl)benzyle** | 70% | 1055.42 | $C_{59}H_{105}N_4O_6Y$ 1055.7 $[M+H]^+$ |
| **C10** | **H3** | **-N-(3,5-di-tert-butyl)benzyle** | 85% | 971,3 | $C_{53}H_{93}N_4O_6Y$ 971.6 $[M+H]^+$ |
| | **H4** | **-N-[3,5-Bis(trifluométhyl] benzyle** | 80% | 994,5 | $C_{47}H_{75}F_6N_4O_6Y$ 995,46 $[M+H]^+$ |
| | **H5** | **-N-[4-(2-PhenylEthyl)-benzyl]** | - | 963.20 | $C_{53}$H85N4O6Y 963.6 $[M+H]^+$ |
| | **H7** | **-N-(4-n-octyl)benzyle** | 82% | 971.26 | $C_{53}H_{93}N_4O_6Y$ 971.6 $[M+H]^+$ |
| **C8** | **H8** | **-N-(4-n-octyl)benzyle** | 93% | 887,1 | $C_{47}H_{81}N_4O_6Y$ 887.5 $[M+H]^+$ |
| | **H9** | **-N-[4-(2-PhenylEthyl)-benzyl]** | 64% | 879.0 | $C_{47}H_{73}N_4O_6Y$ 879.42 $[M+H]^+$ |
| Colonne Symmetry Sunfire™ C-18, 3.5μm, 150x4.6 mm | | | | | |

**IV. Radiochimie :**

Pour le radiomarquage, les matériels suivants ont été utilisés (*Tableau 9*) :

| | | |
|---|---|---|
| **Flacons** | 12 mL en verre borosilicaté serti Elu-III | IBA |
| **Incubateur** | Fisherbrand 15L | Fisher |
| **Tubes** | 5 mL en PP à bouchon à vis blanc | VWR |
| **Seringues** | 1 mL BD Plastipak® | Becton-Dickinson |
| **Aiguilles** | BD Microlance 21G 2" | Becton-Dickinson |
| **Activimètre** | CRC-127R | Capintec |

(suite)

| | | |
|---|---|---|
| **Compteur** | Cobra II Auto-gamma | Packard |

Les expériences ont été réalisées dans des flacons en verre borosilicaté sertis. Les flacons ont été chauffés dans un bloc chauffant Bioblock permettant de chauffer jusqu'à 6 flacons. Lorsqu'une agitation était nécessaire, un vortex Lab Dancer S40 (VWR) a été utilisé. Les centrifugations ont été réalisées avec une centrifugeuse MF 20-R (Awel).

Les activités étaient mesurées dans un activimètre CRC-127R (Capintec), dont la calibration était réalisée chaque matin.

Les contrôles qualité ont été réalisés par CCM sur papier Whatman 1, avec comme éluant un mélange MeOH/$NEt_3$ 0.1 %. Les Puretés RadioChimiques (PRC) sont déterminées à l'aide d'un phosphoimageur Cyclone (Perkin Elmer), et traitées avec le logiciel Optiquant.

**Exemple 8 : Procédure générale pour la complexation de G avec l'yttrium 90**

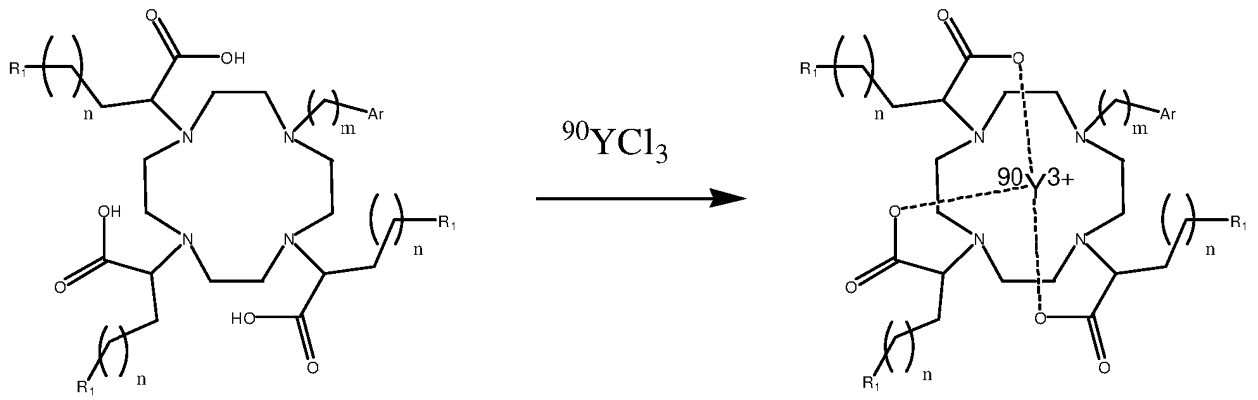


G J

1 mL de chlorure d'yttrium-90 en solution dans un tampon acétate pH = 7 est ajouté à 1 mL de ligand G en solution dans l'éthanol à une concentration de $10^{-3}$ mole/L. La solution est chauffée 30 min à 90°C. Le complexe d'yttrium 90 J est ainsi obtenu.

*Tableau 11 : Rendement de radiomarquage*

| **Rendement de radiomarquage %** | | | | | | | | | | | | | |
|---|---|---|---|---|---|---|---|---|---|---|---|---|---|
| Ligands | G1iso1 | G1iso2 | G1iso3 | G1iso4 | G2 | G3 | G4 | G5 | G6 | G7 | G8 | G9 | G10 |
| Complexes $^{90}$Y J | 89 | 94.4 | 81.5 | 95.5 | 14.1 | 97.4 | 96 | 98.7 | 97.9 | 36.3 | 78.8 | 82.3 | 90 |

| Ligands | G11 | G12 |
|---|---|---|
| Complexes $^{90}$Y J | 76.5 | 35.7 |

Les résultats sont également présentés en Figure 1.

On constate que les ligands selon l'invention ont un rendement de radiomarquage satisfaisant, voire très bon et notamment supérieur à 75%.

**Exemple 9 : Procédure générale pour l'extraction dans l'huile d'œillette Iodée**

2 mL de Lipiodol® sont ajoutés au complexe J préparé à l'exemple 8 et le mélange est agité vigoureusement. Les phases sont ensuite séparées par centrifugation (3500 tours/min, 15 min) et la phase huileuse est collectée pour

donner le radiotraceur attendu H. L'activité de la phase huileuse H est ensuite mesurée pour évaluer le taux d'extraction du complexe radiomarqué J.

*Tableau 12 : Rendement d'extraction du complexe radiomarqué J %*

| Rendement d'extraction du complexe radiomarqué J % | | | | | | | | | | | | | |
|---|---|---|---|---|---|---|---|---|---|---|---|---|---|
| Complexes $^{90}Y$ J | J1iso1 | J1iso2 | J1iso3 | J1iso4 | J2 | J3 | J4 | J5 | J6 | J7 | J8 | J9 | J10 |
| Radiotraceur $^{90}Y$ H | 96.8 | 99.2 | 41.1 | 75.7 | 41.8 | 96.7 | 99.0 | 97.7 | 96.9 | 64.3 | 87.4 | 88.4 | 76.2 |

| Complexes $^{90}Y$ J | J11 | J12 |
|---|---|---|
| Radiotraceur $^{90}Y$ H | 75.9 | 52.2 |

Les résultats sont également présentés en Figure 2.

On constate que les rendements d'extraction des complexes selon l'invention dans le Lipiodol® sont satisfaisants, voire supérieurs à 75% : ils sont donc facilement extractibles dans une phase huileuse.

**Exemple 10 : Procédure générale pour l'évaluation de la stabilité du radiotraceur H**

Le dosage de l'Yttrium-90 relargué dans la solution aqueuse (sérum physiologique ou sérum humain) au cours du temps est réalisé par comptage au compteur gamma. Celui-ci est préalablement calibré pour mesurer l'yttrium-90 (calcul du rendement de comptage de l'appareil pour cet isotope).

A différents temps, un aliquot de 100 μL est prélevé sur chaque échantillon et déposé dans un tube de 5 mL, préalablement pesé. Les tubes sont pesés puis mis à compter au compteur gamma. Les mesures obtenues sont corrigées du rendement de comptage et de la décroissance de l'Yttrium-90 pour déterminer le pourcentage d'Yttrium-90 relargué dans la phase aqueuse, par rapport à l'activité initiale présente dans la solution. Il a également été tenu compte de la masse de liquide prélevée.

1 mL de radiotraceur H fraichement préparé est prélevé puis déposé dans un flacon en verre à fond plat de 12 mL. L'activité est mesurée à l'activimètre, et l'heure est notée. 10 mL de solution saline à 0,9% (sérum physiologique) sont ajoutés et le mélange est agité. Le flacon est ensuite déposé dans l'incubateur placé à 37°C, muni d'un agitateur réglé à 30 rpm.

On laisse l'agitation pendant plusieurs jours. La phase aqueuse est prélevée à différents temps pour doser l'yttrium-90 relargué.

*Tableau 13 : Stabilité à 7 ou 8 j dans le sérum physiologique*

| Test de stabilité 7 j Sérum physiologique | | | | | | | | | | | |
|---|---|---|---|---|---|---|---|---|---|---|---|
| Radiotraceur | H1iso1 | H1iso2 | H1iso3 | H1iso4 | H3 | H5 | H6 | H7 | H8 | H9 | H10 |
| % 7j | 6.8 | 4.15 | 14.7 | 15.7 | 9.8 | 3 | 0.5 | 4.4 | 2.4 | 9.5 | 9 |

| Radiotraceur | H11 | H12 |
|---|---|---|
| % 8j | 19.7 | 8.6 |

Les résultats sont également présentés en Figure 3.

**Exemple 11 : Test de stabilité dans le sérum physiologique à 15 jours**

1 mL de radiotraceur H fraichement préparé est prélevé puis déposé dans un flacon en verre à fond plat de 12 mL. L'activité est mesurée à l'activimètre, et l'heure est notée. 10 mL de solution saline à 0,9% (sérum physiologique) sont ajoutés et le mélange est agité. Le flacon est ensuite déposé dans l'incubateur placé à 37°C, muni d'un agitateur réglé à 30 rpm.
On laisse l'agitation pendant 15 jours. La phase aqueuse est prélevée à différents temps pour doser l'yttrium-90 relargué. Les résultats sont présentés dans le Tableau 14 ci-dessous ainsi qu'en Figure 4.

*Tableau 14 : Stabilité à 15 j dans le sérum physiologique*

| Temps (heure) | H5 % | H6 % |
|---|---|---|
| 1 | 0.5 | 1.56 |
| 24 | 0.27 | 1.52 |
| 48 | 1.5 | 1.23 |
| 72 | 1.06 | 0.57 |
| 138 | 0.71 | 0.71 |
| 168 | 1.07 | 0.61 |
| 216 | 2.03 | 0.74 |
| 240 | 3.32 | 0.96 |
| 360 | 17.67 | 4.9 |

**Exemple 12 : Test de stabilité dans le sérum humain à 15 jours**

1 mL de radiotraceur H fraichement préparé est prélevé puis déposé dans un flacon en verre à fond plat de 12 mL. L'activité est mesurée à l'activimètre, et l'heure est notée. 10 mL de sérum humain sont ajoutés et le mélange est agité. Le flacon est ensuite déposé dans l'incubateur placé à 37°C, muni d'un agitateur réglé à 30 rpm. On laisse l'agitation pendant 15 jours. La phase aqueuse est prélevée à différents temps pour doser l'yttrium-90 relargué.

Les résultats sont présentés dans le Tableau 15 ci-dessous ainsi qu'en Figure 5.

*Tableau 15: Stabilité à 15j dans le sérum humain*

| Temps (heure) | H5 % | H6 % |
|---|---|---|
| 1 | 14,18 | 7,73 |
| 24 | 2,4 | 7,7 |
| 48 | 1,41 | 3,17 |
| 72 | 1 | 2,1 |
| 138 | 4,18 | 7,98 |
| 168 | 6,69 | 10,15 |
| 216 | 8,83 | 12,95 |
| 240 | 9,64 | 14,26 |
| 360 | 19,44 | 18,5 |

En conclusion des tests de stabilité, on constate que les complexes selon l'invention sont stables dans une phase huileuse et ne fuient pas dans une phase aqueuse telle que le sérum physiologique. Cette stabilité permet notamment une excellente vectorisation des complexes lorsqu'administrés dans une phase huileuse telle que le Lipiodol® à des patients.

## Revendications

**1.** Composé de formule générale (I) suivante :

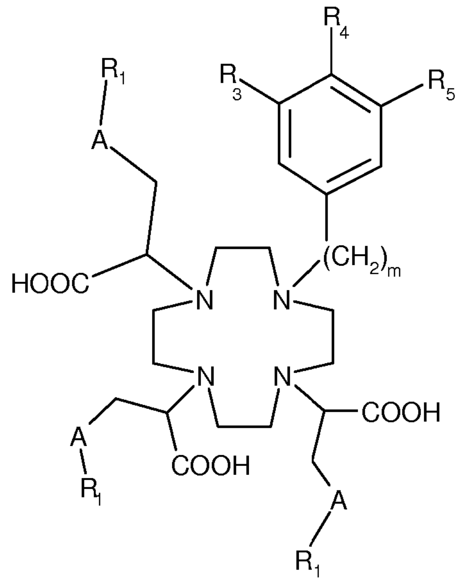

(I)

dans laquelle :

- $R_1$ est un méthyle ou un $(C_6-C_{10})$aryle ;
- $R_3$, $R_4$ et $R_5$ sont choisis indépendamment les uns des autres parmi le groupe constitué de : H, $(C_1-C_{20})$alkyle, $(C_2-C_{20})$alcényle, $(C_2-C_{20})$alcynyle, $(C_6-C_{10})$aryle, $(C_1-C_{20})$alkylène-$(C_6-C_{10})$aryle, $(C_2-C_{20})$alcénylène-$(C_6-C_{10})$aryle et $(C_2-C_{20})$alcynylène-$(C_6-C_{10})$aryle ;
lesdits groupements alkyle, alcényle, alcynyle, alkylène, alcénylène et alcynylène des radicaux $R_3$, $R_4$ et $R_5$ pouvant éventuellement comprendre un ou plusieurs $(C_6-C_{10})$arylène(s) et/ou un ou plusieurs $(C_5-C_{10})$cycloalkylène(s) dans leur chaîne;
et
lesdits groupements alkyle, alcényle, alcynyle, aryle, alkylène, alcénylène et alcynylène des radicaux $R_3$, $R_4$ et $R_5$ étant éventuellement substitué(s) par un ou plusieurs substituant(s) choisis parmi le groupe constitué de : halogène, halogéno$(C_1-C_{20})$alkyle, $(C_1-C_{20})$alkyle, $(C_2-C_{20})$alcényle, $(C_2-C_{20})$alcynyle ; lesdits groupements alkyle, alcényle et alcynyle, pouvant éventuellement comprendre un ou plusieurs $(C_6-C_{10})$arylène(s) dans leur chaîne ;
- A est un groupement $-(CH_2)_n-$ pouvant éventuellement comprendre un ou plusieurs $(C_6-C_{10})$arylène(s) dans sa chaîne ;
- n est un nombre entier allant de 0 à 15 ; et
- m est un nombre entier allant de 1 à 10 ;

ou l'un de ses sels pharmaceutiquement acceptables ou l'un de ses isomères optiques ou l'un de ses isomères géométriques ou l'un de ses tautomères ou l'un de ses solvates.

**2.** Composé de formule (I) selon la revendication 1, dans laquelle lorsque $R_1$ est un méthyle, alors n est un nombre entier allant de 4 à 8.

**3.** Composé selon la revendication 1, de formule générale (I-1) suivante :

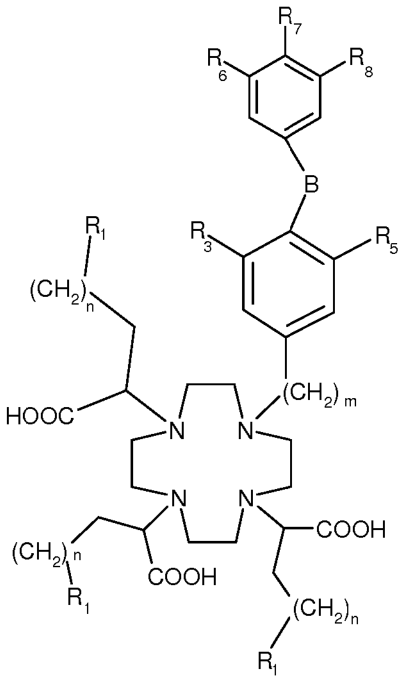

(I-1)

dans laquelle :

- $R_1$, $R_3$, $R_5$, n et m sont tels que définis à la revendication 1, avec n étant de préférence égal à 6 ou égal à 8 ;
- B est une liaison, un ($C_1$-$C_{20}$)alkylène, un ($C_2$-$C_{20}$)alcénylène ou un ($C_2$-$C_{20}$)alcynylène ; et
- $R_6$, $R_7$ et $R_8$ sont choisis, indépendamment les uns des autres, parmi H et ($C_1$-$C_{20}$)alkyle.

**4.** Composé selon la revendication 1, de formule générale (I-2) ou (I-3) suivante :

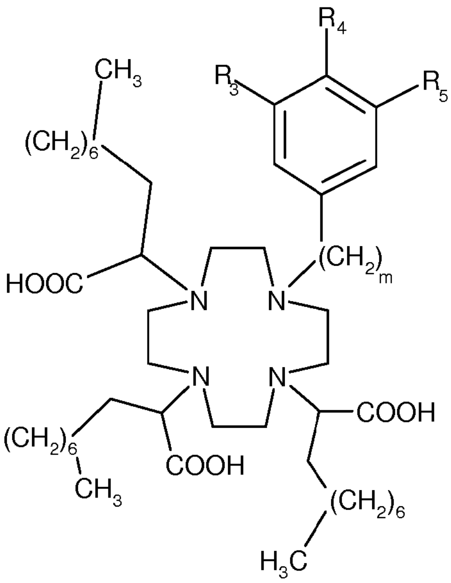

(I-2)

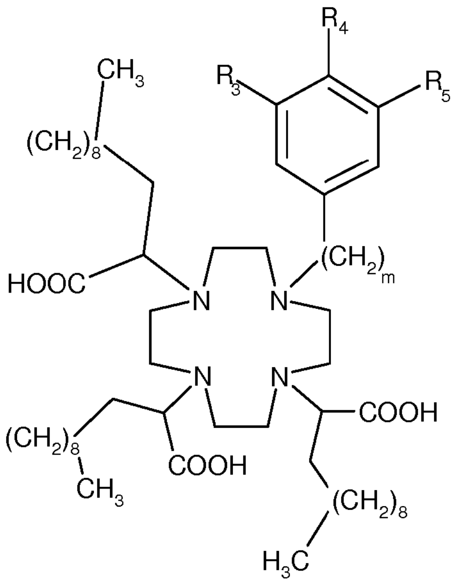

(I-3)

dans laquelle $R_3$, $R_4$, $R_5$ et m sont tels que définis à la revendication 1.

**5.** Composé de formule (I) selon la revendication 1, choisi parmi le groupe constitué des composés suivants :

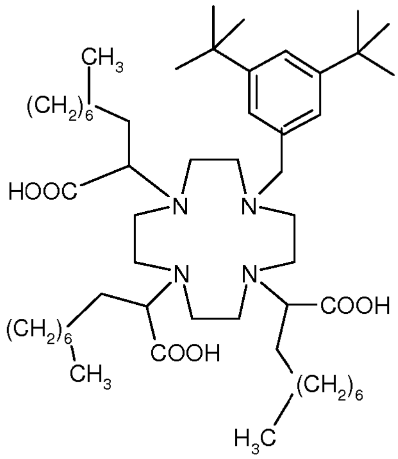

G3

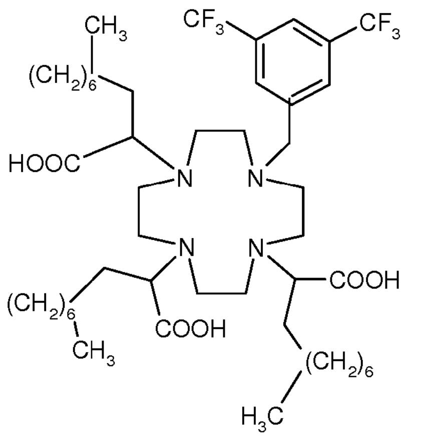

G4

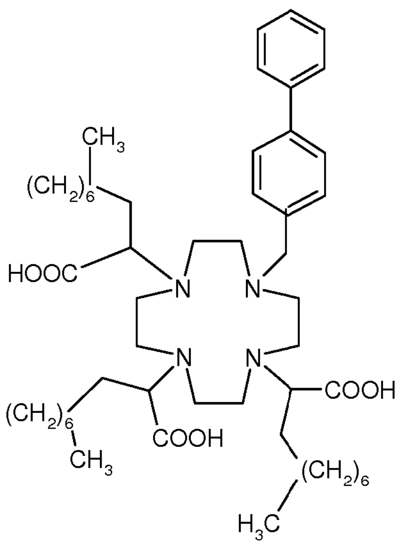

G6

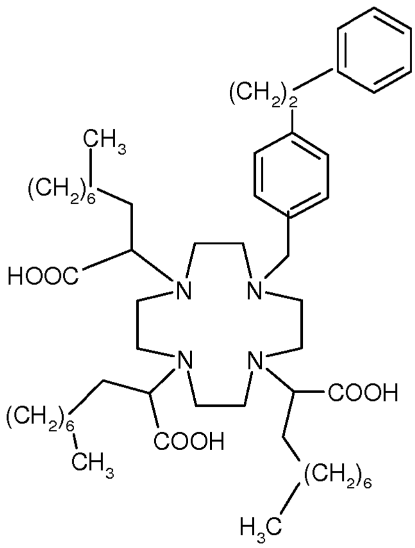

G5

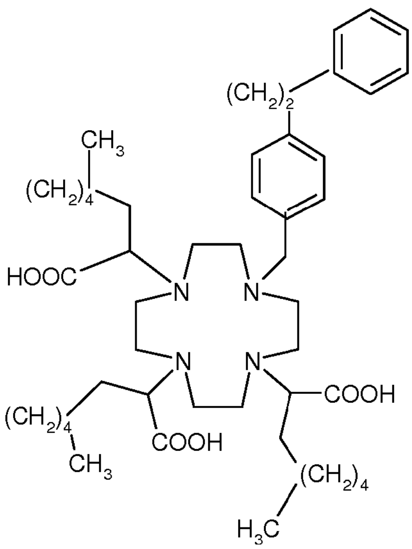

G9

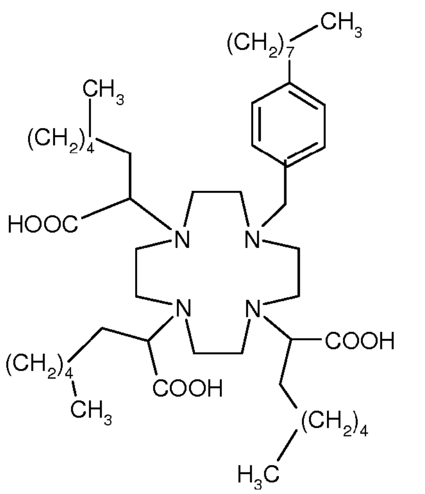

G8

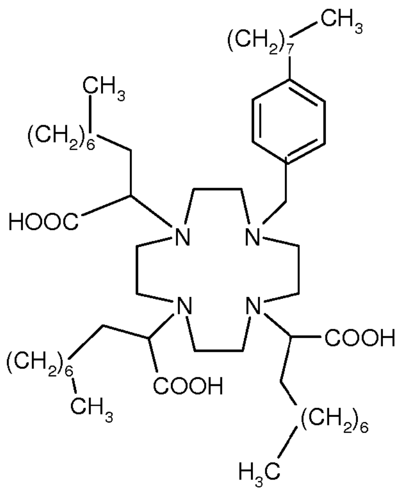

G7

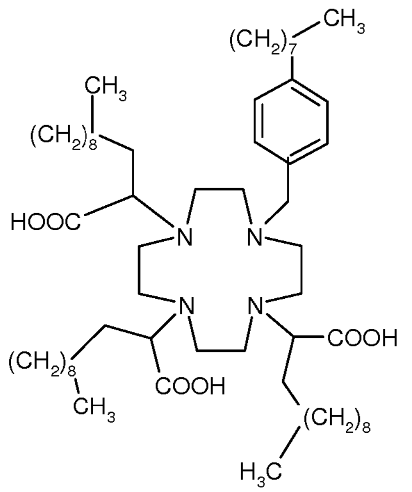

G2

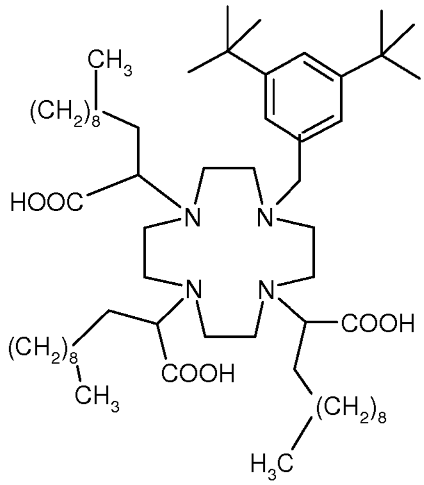

G1

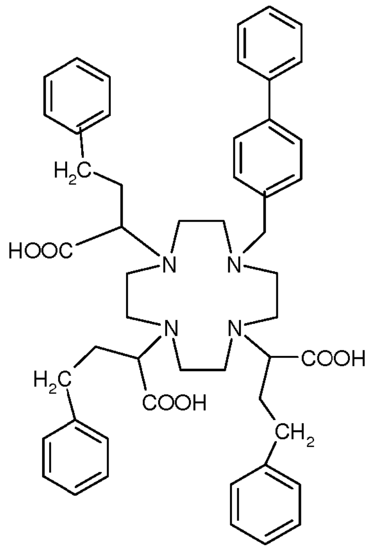

G10

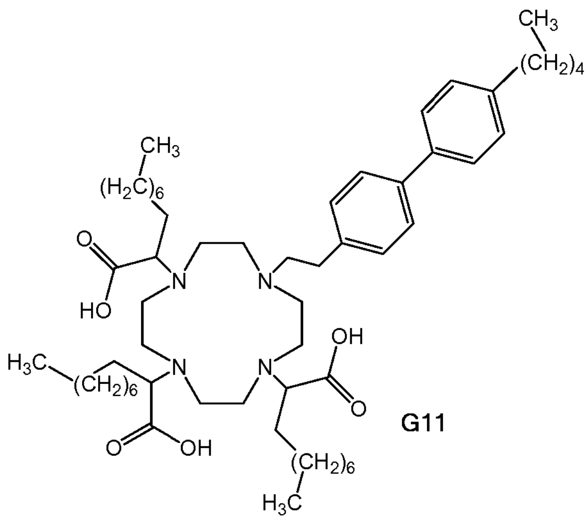

G11

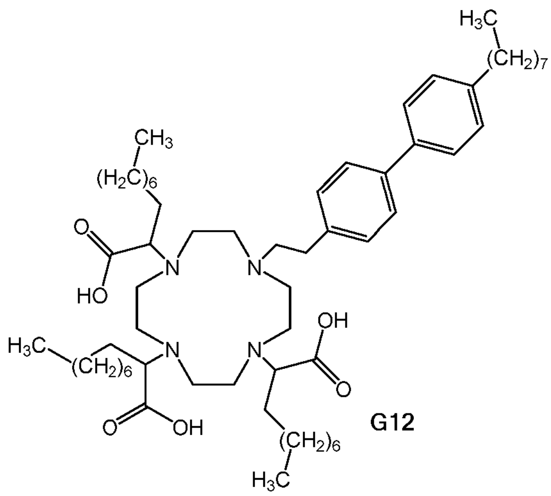

G12

ou leurs sels pharmaceutiquement acceptables.

**6.** Complexe d'un composé de formule (I) ou d'un de ses sels selon l'une quelconque des revendications 1 à 5, avec M ; M étant un élément chimique, de préférence un radioélément.

**7.** Complexe selon la revendication 6, pour son utilisation dans le traitement des cancers, en particulier des cancers du foie.

**8.** Composition pharmaceutique comprenant un composé selon l'une quelconque des revendications 1 à 5 ou un complexe selon l'une quelconque des revendications 6 à 7 et éventuellement un ou plusieurs excipient(s) pharmaceutiquement acceptable(s).

**9.** Composition pharmaceutique selon la revendication 8, comprenant en outre une huile iodée, notamment une huile iodée comprenant des esters éthyliques d'acides gras iodés de l'huile d'œillette.

## Patentansprüche

**1.** Verbindung der folgenden allgemeinen Formel (I):

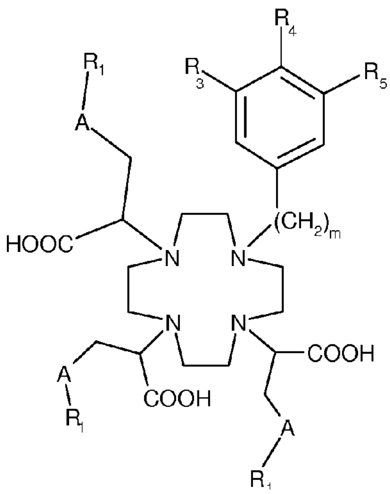

(I)

in der:

- $R_1$ für ein Methyl oder ein $(C_6\text{-}C_{10})$-Aryl steht;
- $R_3$, $R_4$ und $R_5$ unabhängig voneinander aus der Gruppe bestehend aus H, $(C_1\text{-}C_{20})$-Alkyl, $(C_2\text{-}C_{20})$-Alkenyl, $(C_2\text{-}C_{20})$-Alkinyl, $(C_6\text{-}C_{10})$-Aryl, $(C_1\text{-}C_{20})$-Alkylen- $(C_6\text{-}C_{10})$-aryl, $(C_2\text{-}C_{20})$-Alkenylen-$(C_6\text{-}C_{10})$-aryl und $(C_2\text{-}C_{20})$-Alkinylen- $(C_6\text{-}C_{10})$-aryl ausgewählt sind;

  wobei die Alkyl-, Alkenyl-, Alkinyl-, Alkylen-, Alkenylen- und Alkinylengruppen der Reste $R_3$, $R_4$ und $R_5$ gegebenenfalls eine oder mehrere $(C_6\text{-}C_{10})$-Arylengruppen und/oder eine oder mehrere $(C_5\text{-}C_{10})$-Cycloalkylengruppen in ihrer Kette umfassen können und

  wobei die Alkyl-, Alkenyl-, Alkinyl-, Alkylen-, Alkenylen- und Alkinylengruppen der Reste $R_3$, $R_4$ und $R_5$ gegebenenfalls durch einen oder mehrere Substituenten aus der Gruppe bestehend aus Halogen, Halogen- $(C_1\text{-}C_{20})$ -alkyl, $(C_1\text{-}C_{20})$ -Alkyl, $(C_2\text{-}C_{20})$-Alkenyl und $(C_2\text{-}C_{20})$-Alkinyl substituiert sein können; wobei die Alkyl-, Alkenyl- und Alkinylgruppen gegebenenfalls eine oder mehrere $(C_6\text{-}C_{10})$-Arylengruppen in ihrer Kette umfassen können;
- A für eine Gruppe $-(CH_2)_n-$ steht, die gegebenenfalls eine oder mehrere $(C_6\text{-}C_{10})$-Arylengruppen in ihrer Kette umfassen kann;
- n für eine ganze Zahl im Bereich von 0 bis 15 steht und
- m für eine ganze Zahl im Bereich von 1 bis 10 steht;

oder ein pharmazeutisch unbedenkliches Salz davon oder ein optisches Isomer davon oder ein geometrisches Isomer davon oder ein Tautomer davon oder ein Solvat davon.

**2.** Verbindung der Formel (I) nach Anspruch 1, wobei dann, wenn $R_1$ für ein Methyl steht, n für eine ganze Zahl im Bereich von 4 bis 8 steht.

**3.** Verbindung nach Anspruch 1 der folgenden allgemeinen Formel (1-1):

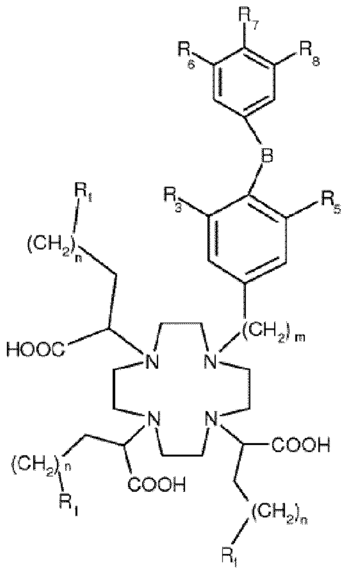

(I-1)

in der:

- $R_1$, $R_3$, $R_5$, n und m wie in Anspruch 1 definiert sind, wobei n vorzugsweise gleich 6 oder gleich 8 ist;
- B für eine Bindung, ein ($C_1$-$C_{20}$)-Alkylen, ein ($C_2$-$C_{20}$) -Alkenylen oder ein ($C_2$-$C_{20}$) -Alkinylen steht und
- $R_6$, $R_7$ und $R_8$ unabhängig voneinander aus H und ($C_1$-$C_{20}$) -Alkyl ausgewählt sind.

**4.** Verbindung nach Anspruch 1 der folgenden allgemeinen Formel (I-2) oder (I-3):

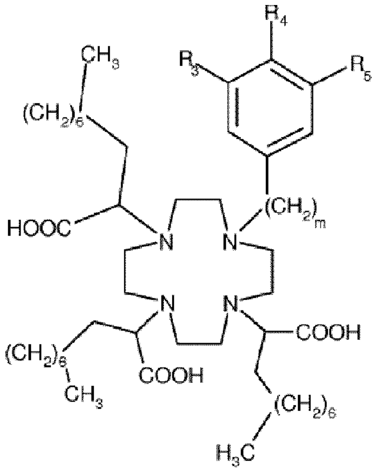

(I-2)

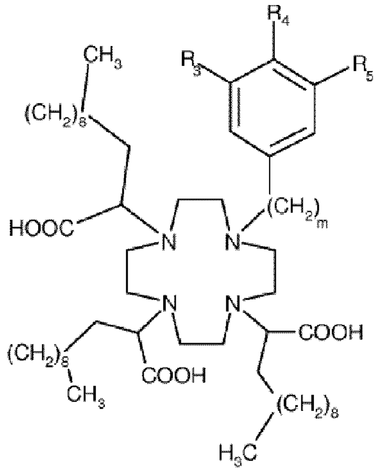

(I-3)

in der $R_3$, $R_4$, $R_5$ und m wie in Anspruch 1 definiert sind.

**5.** Verbindung der Formel (I) nach Anspruch 1, die aus der Gruppe bestehend aus den folgenden Verbindungen ausgewählt ist:

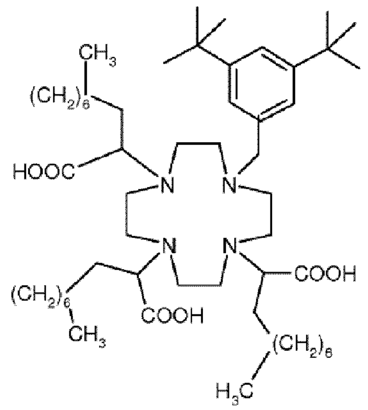

G3

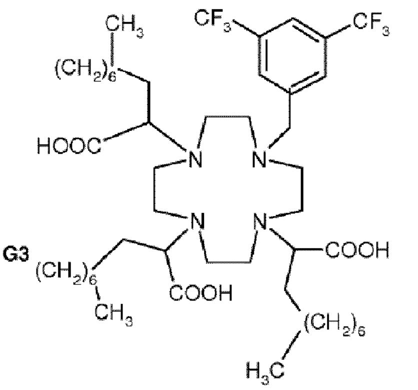

G4

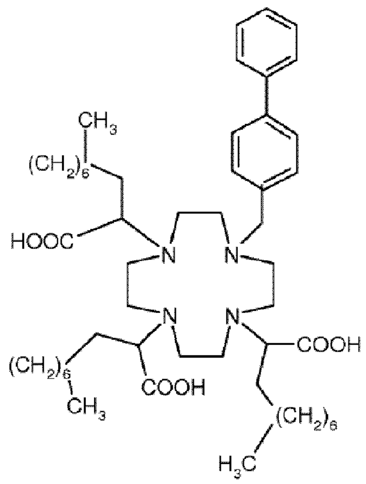

G6

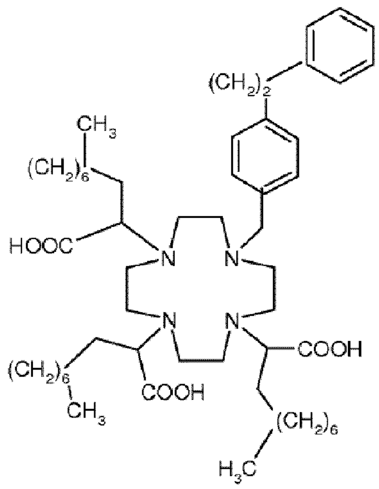

G5

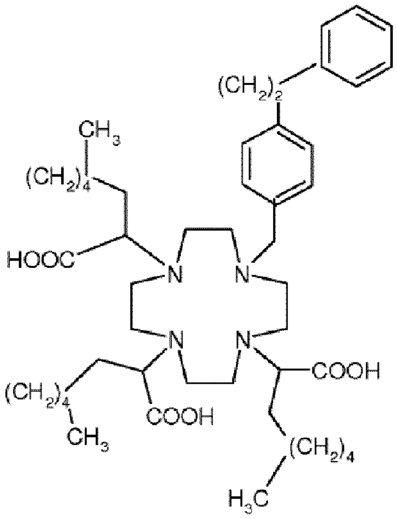

G9

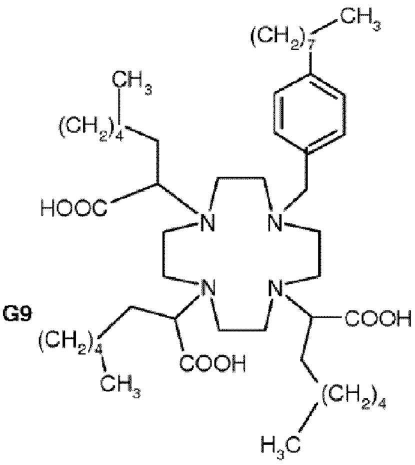

G8

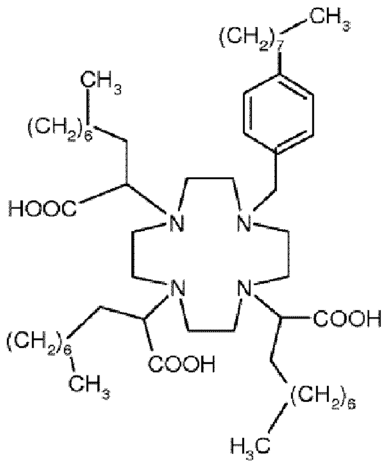
G7

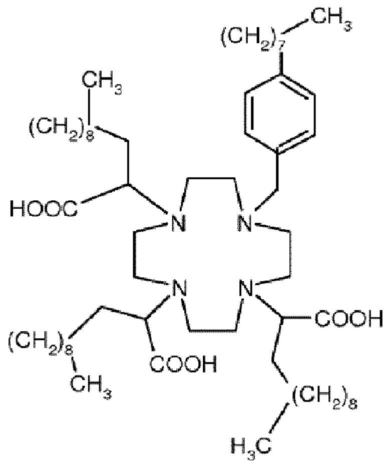
G2

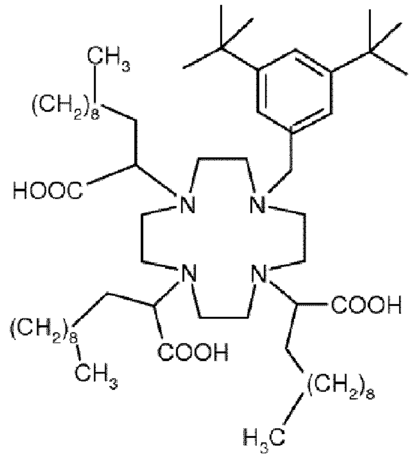
G1

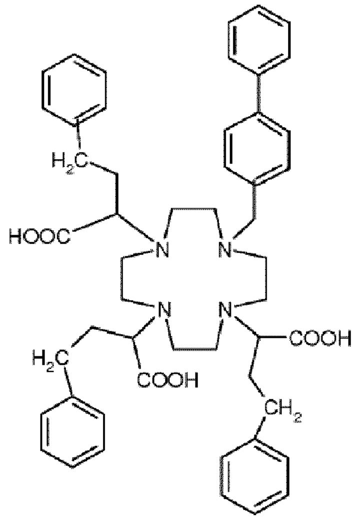
G10

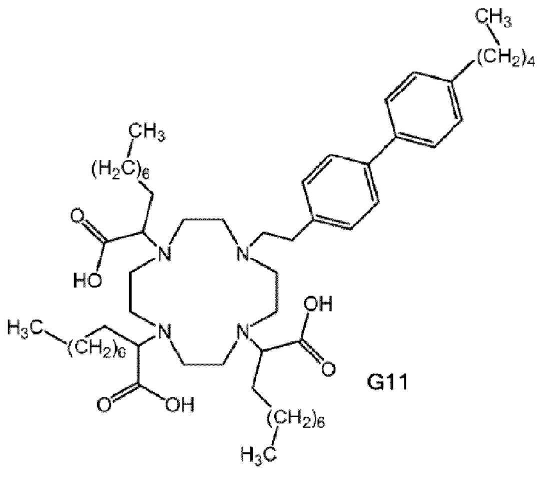
G11

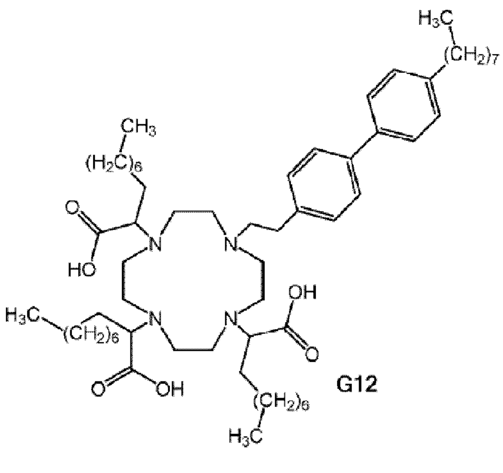
G12

oder pharmazeutisch unbedenkliche Salze davon.

**6.** Komplex einer Verbindung der Formel (I) oder eines Salzes davon nach einem der Ansprüche 1 bis 5 mit M; wobei M für ein chemisches Element, vorzugsweise ein Radioelement, steht.

**7.** Komplex nach Anspruch 6 zur Verwendung bei der Behandlung von Krebserkrankungen, insbesondere Leberkrebserkrankungen.

8. Pharmazeutische Zusammensetzung, umfassend eine Verbindung nach einem der Ansprüche 1 bis 5 oder einen Komplex nach einem der Ansprüche 6 bis 7 und gegebenenfalls einen oder mehrere pharmazeutisch unbedenkliche Hilfsstoffe.

9. Pharmazeutische Zusammensetzung nach Anspruch 8, ferner umfassend ein iodiertes Öl, insbesondere ein iodiertes Öl, das Ethylester von iodierten Mohnölfettsäuren umfasst.

**Claims**

1. Compound of the following general formula (I):

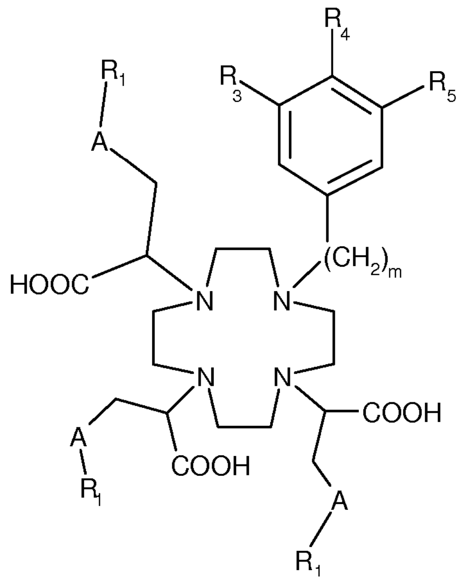

(I)

in which:

- $R_1$ is a methyl or a $(C_6\text{-}C_{10})$ aryl;
- $R_3$, $R_4$ and $R_5$ are selected independently of one another from the group consisting of: H, $(C_1\text{-}C_{20})$ alkyl, $(C_2\text{-}C_{20})$ alkenyl, $(C_2\text{-}C_{20})$ alkynyl, $(C_6\text{-}C_{10})$ aryl, $(C_1\text{-}C_{20})$ alkylene- $(C_6\text{-}C_{10})$ aryl, $(C_2\text{-}C_{20})$ alkenylene- $(C_6\text{-}C_{10})$ aryl and $(C_2\text{-}C_{20})$ alkynylene- $(C_6\text{-}C_{10})$ aryl;
said alkyl, alkenyl, alkynyl, alkylene, alkenylene and alkynylene groups of the radicals $R_3$, $R_4$ and $R_5$ optionally comprising one or more $(C_6\text{-}C_{10})$ arylene (s) and/or one or more $(C_5\text{-}C_{10})$ cycloalkylene (s) in their chain; and said alkyl, alkenyl, alkynyl, aryl, alkylene, alkenylene and alkynylene groups of the radicals $R_3$, $R_4$ and $R_5$ optionally being substituted with one or more substituent(s) selected from the group consisting of: halogen, halo $(C_1\text{-}C_{20})$ alkyl, $(C_1\text{-}C_{20})$ alkyl, $(C_2\text{-}C_{20})$ alkenyl, $(C_2\text{-}C_{20})$alkynyl; said alkyl, alkenyl and alkynyl groups optionally comprising one or more $(C_6\text{-}C_{10})$ arylene (s) in their chain;
- A is a group $-(CH_2)_n-$ which may optionally comprise one or more $(C_6\text{-}C_{10})$arylene(s) in its chain;
- n is an integer in the range from 0 to 15; and
- m is an integer in the range from 1 to 10;

or a pharmaceutically acceptable salt thereof or an optical isomer thereof or a geometric isomer thereof or a tautomer thereof or a solvate thereof.

2. Compound of formula (I) according to Claim 1, in which when $R_1$ is a methyl, n is an integer in the range from 4 to 8.

3. Compound according to Claim 1, of the following general formula (I-1):

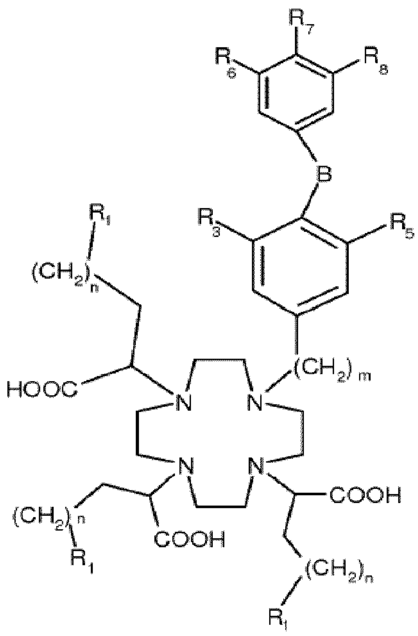

(I-1)

in which:

- $R_1$, $R_3$, $R_5$, n and m are as defined in Claim 1, with n preferably being equal to 6 or equal to 8;
- B is a bond, a $(C_1\text{-}C_{20})$alkylene, a $(C_2\text{-}C_{20})$alkenylene or a $(C_2\text{-}C_{20})$alkynylene; and
- $R_6$, $R_7$ and $R_8$ are selected, independently of one another, from H and $(C_1\text{-}C_{20})$ alkyl.

**4.** Compound according to Claim 1, of the following general formula (1-2) or (1-3):

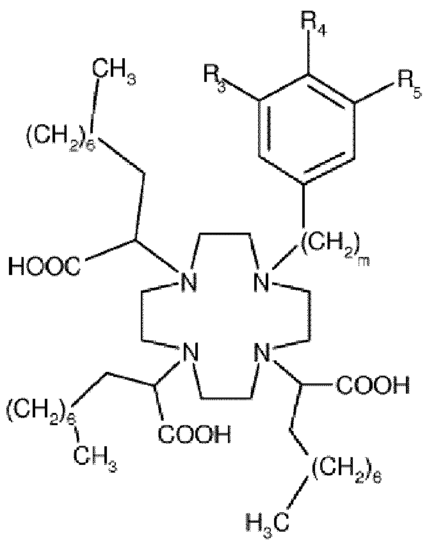

(I-2)

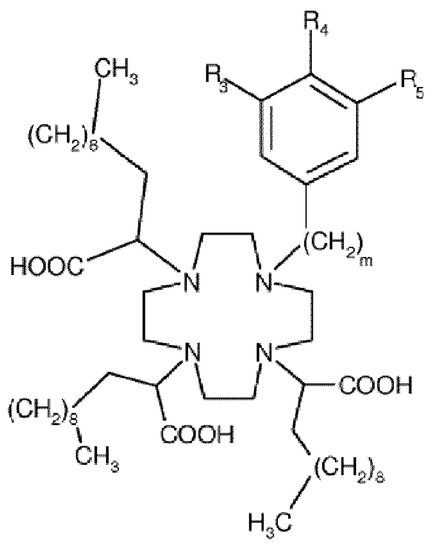

(I-3)

in which $R_3$, $R_4$, $R_5$ and m are as defined in Claim 1.

**5.** Compound of formula (I) according to Claim 1, selected from the group consisting of the following compounds:

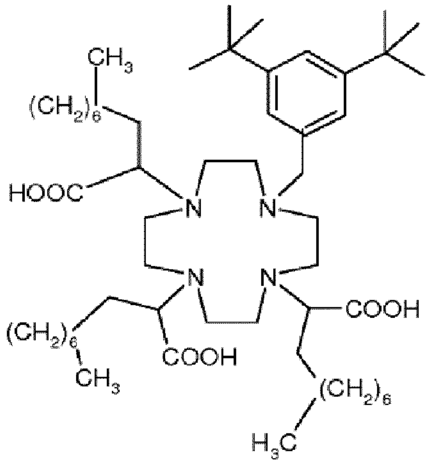 G3 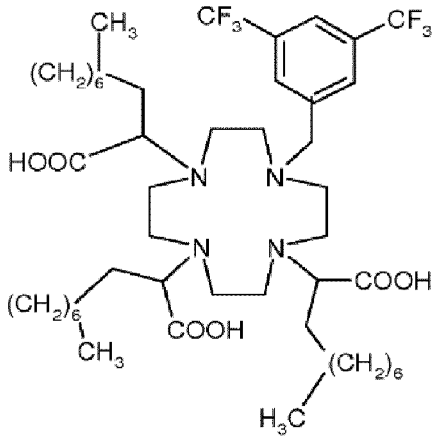 G4

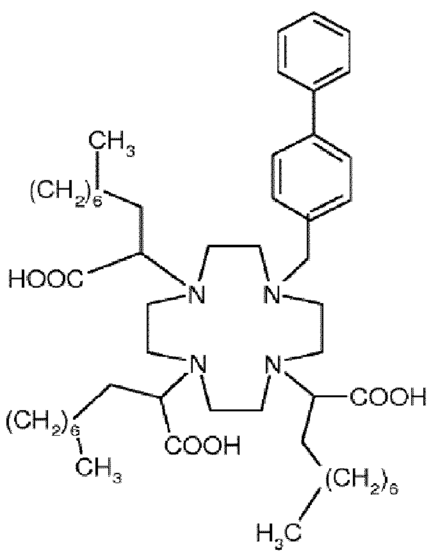 G6 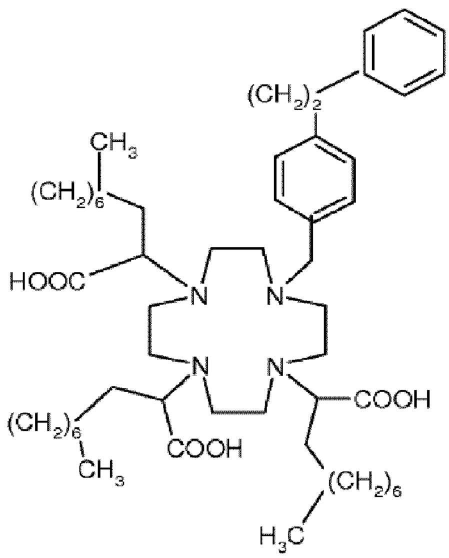 G5

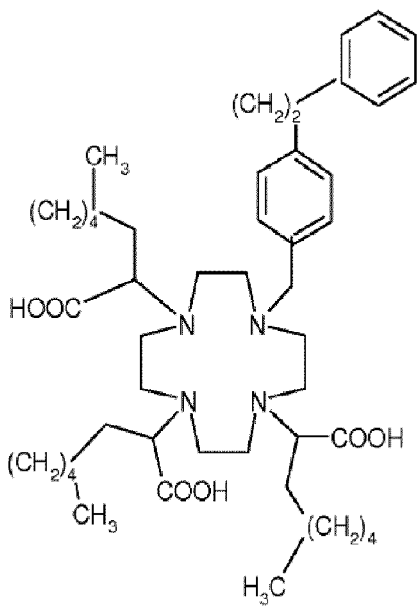 G9 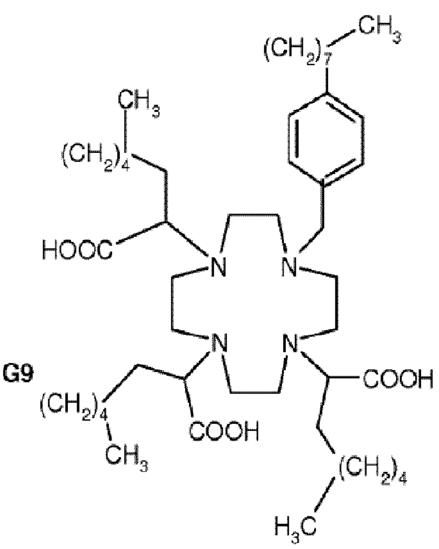 G8

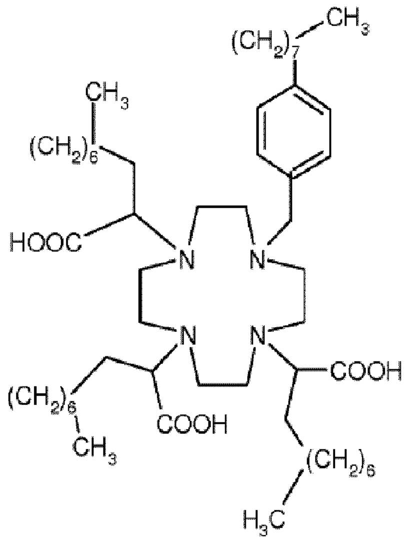

G7

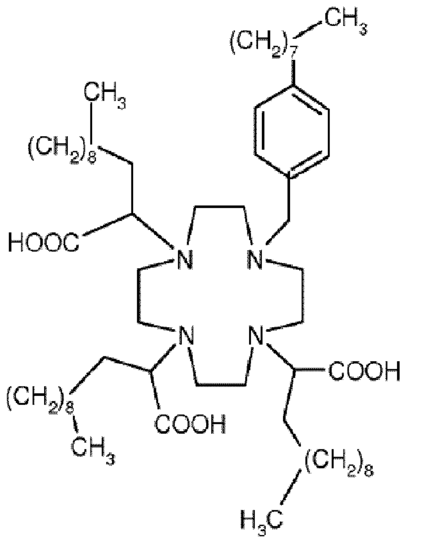

G2

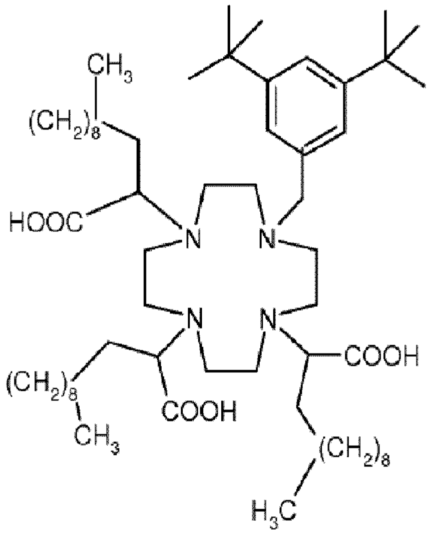

G1

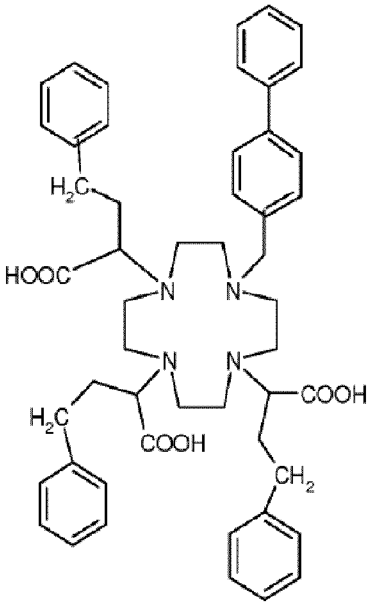

G10

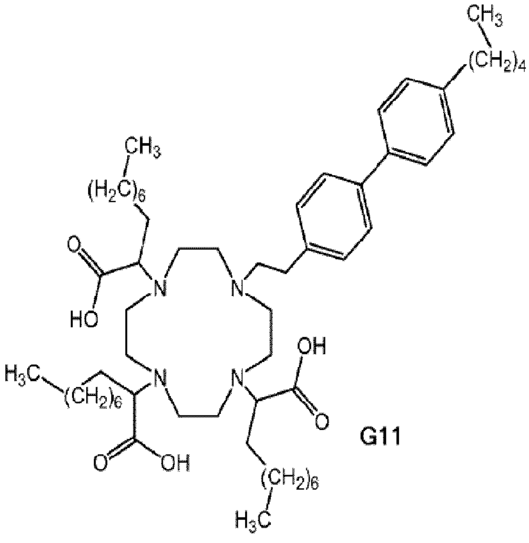

G11

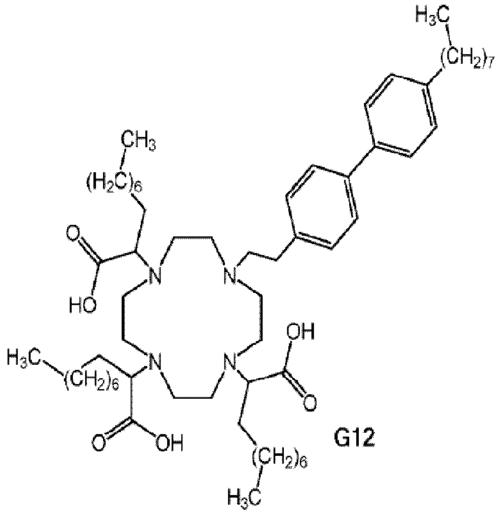

G12

or their pharmaceutically acceptable salts.

**6.** Complex of a compound of formula (I) or of a salt thereof according to any one of Claims 1 to 5, with M; M being a chemical element, preferably a radioelement.

**7.** Complex according to Claim 6, for use in the treatment of cancers, in particular liver cancers.

**8.** Pharmaceutical composition comprising a compound according to any one of Claims 1 to 5 or a complex according to any one of Claims 6 to 7 and optionally one or more pharmaceutically acceptable excipients.

**9.** Pharmaceutical composition according to Claim 8, further comprising an iodinated oil, notably an iodinated oil comprising iodinated ethyl esters of fatty acids of poppyseed oil.

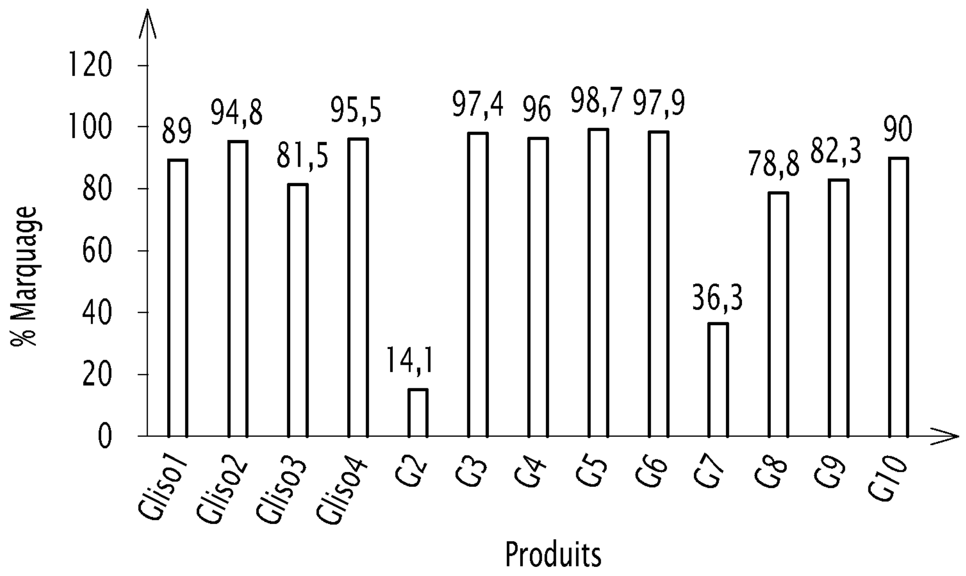
120
100
80
60
40
20
0
% Marquage
89
94,8
81,5
95,5
14,1
97,4
96
98,7
97,9
36,3
78,8
82,3
90
Gliso1
Gliso2
Gliso3
Gliso4
G2
G3
G4
G5
G6
G7
G8
G9
G10
Produits

FIG.1

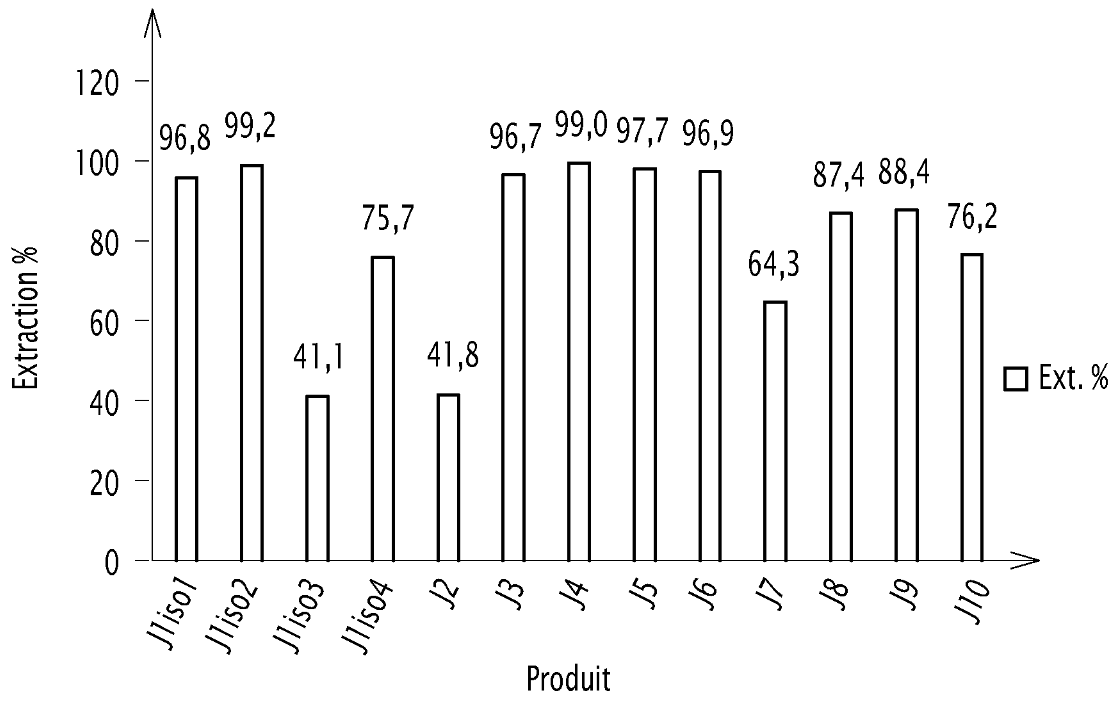
120
100
80
60
40
20
0
Extraction %
96,8
99,2
41,1
75,7
41,8
96,7
99,0
97,7
96,9
64,3
87,4
88,4
76,2
Ext. %
J1iso1
J1iso2
J1iso3
J1iso4
J2
J3
J4
J5
J6
J7
J8
J9
J10
Produit

FIG.2

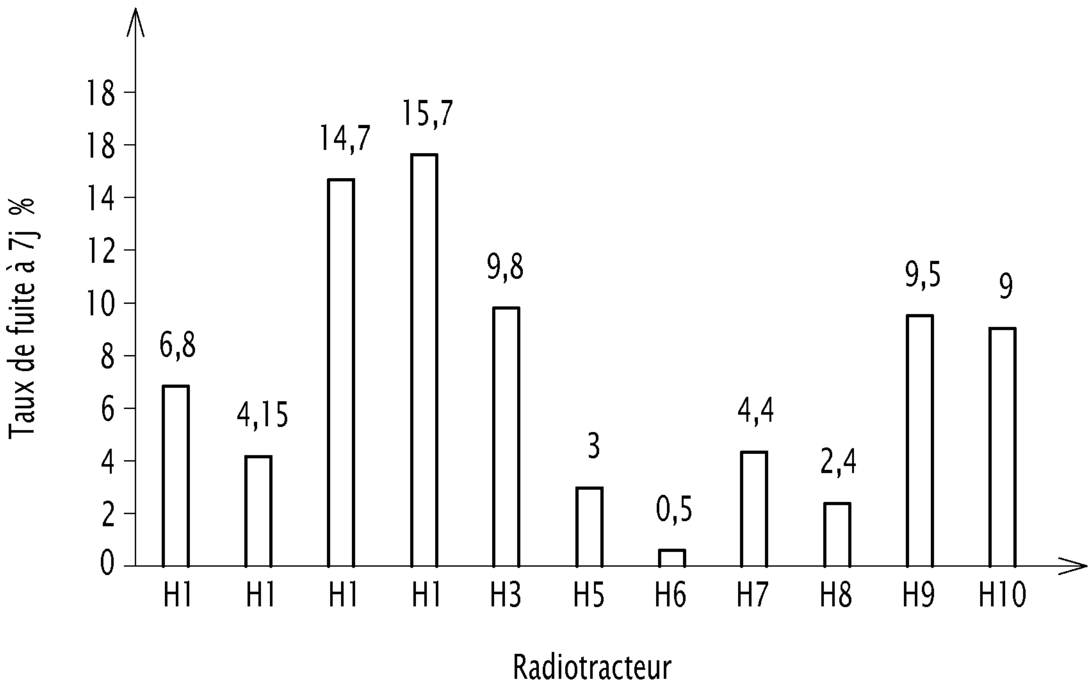
Taux de fuite à 7j %
18
18
14
12
10
8
6
4
2
0
6,8
4,15
14,7
15,7
9,8
3
0,5
4,4
2,4
9,5
9
H1
H1
H1
H1
H3
H5
H6
H7
H8
H9
H10
Radiotracteur

FIG.3

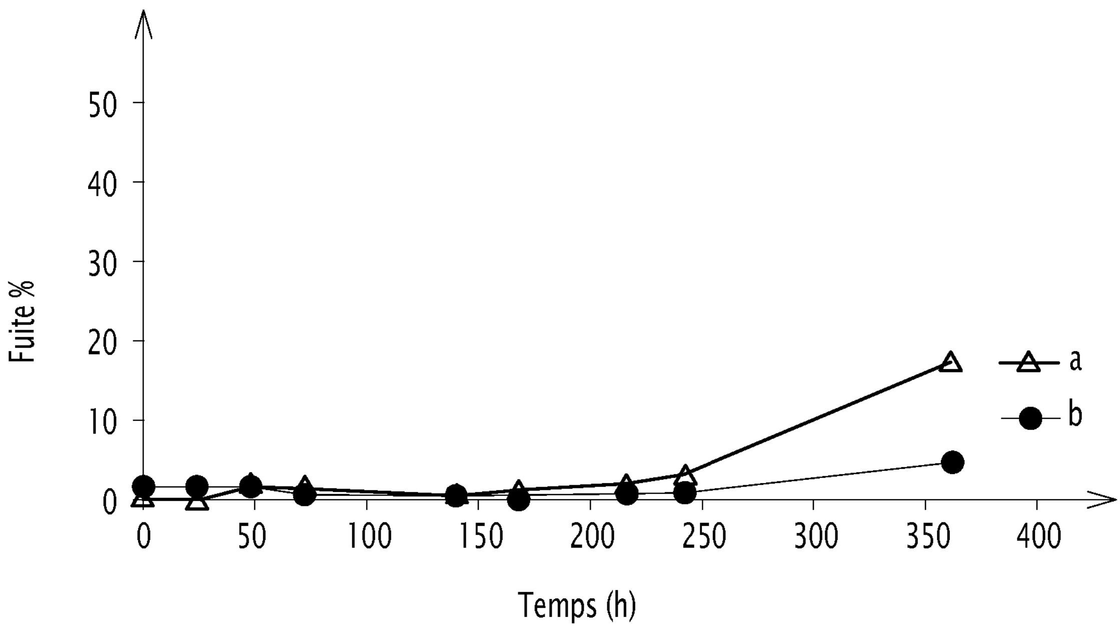
Fuite %
50
40
30
20
10
0
0
50
100
150
200
250
300
350
400
Temps (h)
a
b

FIG.4

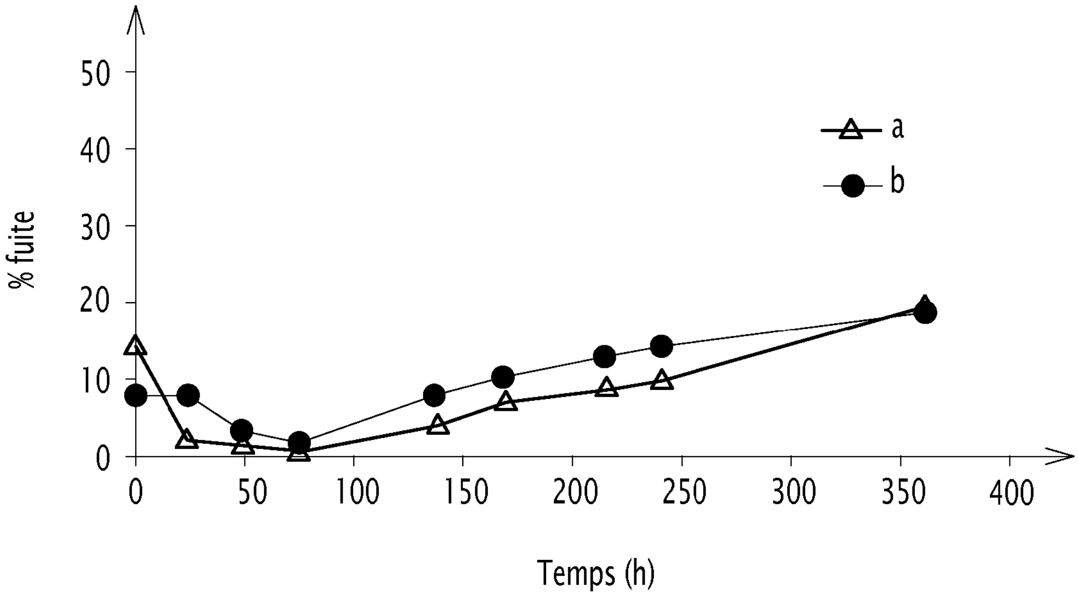


FIG.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03074523 A
- EP 0922700 A


### Littérature non-brevet citée dans la description


- **WOLFF et al.** *Medicine,* 2001, vol. 80, 20-36
- **BERGE et al.** *J. Pharm. Sd,* 1977, vol. 66, 1